# EUROPEAN PATENT APPLICATION

(11) **EP 2 505 661 A1**
(43) Date of publication of application: **03.10.2012**
(21) Application number: 11160096.1
(22) Date of filing: 28.03.2011
(51) Int. Cl.: C12Q 1/52, G01N 33/68, C12Q 1/68

(54) **Methods for detecting the mortality risk**

(71) Applicant: Universitätsklinikum Hamburg-Eppendorf, 20246 Hamburg (DE)
(72) Inventor: Böger, Rainer, 22459 Hamburg (DE); Schwedhelm, Edzard, 20249 Hamburg (DE); Isbrandt, Dirk, 22395 Hamburg (DE); Choe, Chi-Un, 20253 Hamburg (DE); Blankenberg, Stefan, 20251 Hamburg (DE)
(74) Representative: UEXKÜLL & STOLBERG

(57) **Abstract**

The present invention generally relates to methods for determining the mortality risk in a subject. In one aspect, the invention relates to a method for predicting the all-cause mortality risk of a test subject based on the detection of pathologically decreased expression levels of the gene encoding the L-arginine:glycine amidinotransferase (AGAT) and/or pathologically decreased activity levels of the AGAT enzyme. In a further aspect, the invention relates to a method for predicting the all-cause mortality risk of a test subject based on the detection of genetic alterations which are associated with such pathologically decreased expression levels and/or activity levels. Finally, the invention also relates to compounds which are effective in increasing the expression of the AGAT gene and/or the activity of the AGAT enzyme for use in a method of decreasing the mortality risk of a subject.

SEQUENCE LISTING

<110>  Universitätsklinikum Hamburg-Eppendorf

<120>  METHODS FOR DETECTING THE MORTALITY RISK

<130>  P 84722

<160>  18

<170>  PatentIn version 3.3

<210>  1
<211>  52
<212>  DNA
<213>  Homo sapiens

<220>
<221>  variation
<222>  (27)..(27)
<223>  SNP: A replaced by G

<400>  1
acctgcctct ccctgagtct tcctctattt agttaaggta attccatccc tc          52

<210>  2
<211>  52
<212>  DNA
<213>  Homo sapiens

<220>
<221>  variation
<222>  (27)..(27)
<223>  SNP: T replaced by C

<400>  2
agataatttc agatagcaat aagtactaga aaggtgtaaa acttggtaat gg          52

<210>  3
<211>  52
<212>  DNA
<213>  Homo sapiens

<220>
<221>  variation
<222>  (27)..(27)
<223>  SNP: C replaced by T

<400>  3
gcagcccttt tgcaactttt aaacagcgat gagaattctg gccctttgtc tt          52

<210>  4
<211>  52
<212>  DNA
<213>  Homo sapiens

<220>
<221>  variation
<222>  (27)..(27)
<223>  SNP: C replaced by T

<400>  4

caaatgtgta taaatctata ggggatcgta aactgaagga actaatgcct ga 52

<210> 5
<211> 52
<212> DNA
<213> Homo sapiens

<220>
<221> variation
<222> (27)..(27)
<223> SNP: T replaced by C

<400> 5
tttttctcca tttcactgag gttctgtagt aggcaatgct gtcttagcat gt 52

<210> 6
<211> 52
<212> DNA
<213> Homo sapiens

<220>
<221> variation
<222> (27)..(27)
<223> SNP: C replaced by T

<400> 6
ccttctcagt gcacctgatt atgtccttga gtctcagaga ttttttgctt cc 52

<210> 7
<211> 52
<212> DNA
<213> Homo sapiens

<220>
<221> variation
<222> (27)..(27)
<223> SNP: A replaced by T

<400> 7
caccaagcta ggttccagcg taactgatgt gaacctggat attgaactaa cc 52

<210> 8
<211> 52
<212> DNA
<213> Homo sapiens

<220>
<221> variation
<222> (27)..(27)
<223> SNP: C replaced by T

<220>
<221> variation
<222> (27)..(27)
<223> SNP: T replaced by C

<400> 8
gaaaatattt tgctaagcga tagaagttca tcacaaaaga ccacttactg aa 52

<210> 9

<211>  52
<212>  DNA
<213>  Homo sapiens

<220>
<221>  variation
<222>  (27)..(27)
<223>  SNP: C replaced by T

<400>  9
tccacataca cctgatgaaa agcttacgac acaggaagac atcagctcaa ca          52

<210>  10
<211>  52
<212>  DNA
<213>  Homo sapiens

<220>
<221>  variation
<222>  (27)..(27)
<223>  SNP: A replaced by C

<400>  10
tatataccct aatgtgcacc aatccaaaca actggataga tttctcaagc ag          52

<210>  11
<211>  52
<212>  DNA
<213>  Homo sapiens

<220>
<221>  variation
<222>  (27)..(27)
<223>  SNP: A replaced by C

<400>  11
cccaagagaa gtgaaaacat atgcctacgc agaaacttgt atgtaagtgt tc          52

<210>  12
<211>  52
<212>  DNA
<213>  Homo sapiens

<220>
<221>  variation
<222>  (27)..(27)
<223>  SNP: G replaced by T

<400>  12
catttcttga atagatttgt ttgtgtgtat aagctagcca gtggtttagt ta          52

<210>  13
<211>  52
<212>  DNA
<213>  Homo sapiens

<220>
<221>  variation
<222>  (27)..(27)
<223>  SNP: A replaced by G

<400>  13
gtagattaaa taatgttaat ctttccagca tttccatttt tgtgttatgt tc          52

<210>  14
<211>  52
<212>  DNA
<213>  Homo sapiens

<220>
<221>  variation
<222>  (27)..(27)
<223>  SNP: C replaced by T

<400>  14
ggtatcaaaa cctctcattt ctttcactga ggctttaaca gtgctaggct gg          52

<210>  15
<211>  52
<212>  DNA
<213>  Homo sapiens

<220>
<221>  variation
<222>  (27)..(27)
<223>  A replaced by T

<220>
<221>  variation
<222>  (27)..(27)
<223>  SNP: A replaced by T

<400>  15
tgatctttgg gaaaataatg ccctccatgc ttctggtaaa atggccagta ct          52

<210>  16
<211>  24659
<212>  DNA
<213>  Homo sapiens

<400>  16
attgtgaggt caggagttcg agaccagcct ggccaatatg gtgaaacccc atctctacta      60

aaaatacaaa aattagccag aaatcgcttt aacccgggag gcggaggttg cagtgagctg     120

agatcatgcc actgcactcc agcctgggca acagagtgag actacattaa attaaaaaaa     180

aaaaaagcag caatttggca aatgggttgg cctttgcctt taagtttagt cagaacctta     240

agaaagagaa tggtgtttac tgtttttaaa ttccagaagg gtatttgaag actaaagtaa     300

aaaaaataag aagatccctt ggagcagact tcaaatgttt cttcctcagt tttcttaaag     360

ctgtatcagt ttcagtgcta acctaagctg agattatttt aaatacttgg agaaagctgg     420

catttaattt agctggagct agtgttttgc agttgtcttc caatgaaaga atctacctca     480

ttgggagatg ttgagcagta ataacacagc tgttcaaaac tagtaaaaat cctaaaatga     540

ctcgtaaggg acagtgcaga ggctgccttc acaagatttt gtaactcatt tgaaatcaaa     600

gactgcaaag taccaaagag ggagctctgg ttattctagc ctgggccatc agagtggcat     660

tgaacaattg attaaggact ctggctgtgt caggcactgt gctagggata tactggtggg     720

taaaacagac tccctgtcct taaggagcca acagccatat aagcaggcaa ttccagggaa 780
cacaggctat tgtgggatca ggccagtccc acagcatttc agatttagaa agtaaattcc 840
atcagtttac ctggaattga ctaggtagtc aggaaaaaga gaacattaag aagggaaaca 900
taattaggaa gcaagaactg agctagaact caccacactc tgagggataa gctagaatgt 960
ttattattat tattattact aatatttttg agacagagtt tctctcctgt tgcccaggct 1020
ggagtgcagt ggtatgatct cggctcaggg caacctccac ctcctgggtt caagcaattc 1080
tcctgcctca gcctccagag tagctgggat tacaggtgtg caccaacacg cctggctaat 1140
ttttgtattt ttagtagaga cggggttttg ccatatttgc caggctggtc tcagatgggt 1200
tcaagcaatt ctcctgcctc aacctcccga gtagctggga ttacaggtgt gcaccaacac 1260
acctggctaa tttttgtatt tttagtagag atggggtttt gccatgtttg ccaggctggt 1320
ctcaaacttc tgacctcagg tgatccaccc acctcagcct cccagagtgc tgagattaca 1380
ggtgtgaacc accacaccca gcagaatact gatttaatac tttacaatga gatcttcatc 1440
tttccagcta tcagatcact tgtgctcagc ctgcagggta actcccctat gccaaggttc 1500
ccagggatga gcttggtgag ctcacaagtt agaggaggtg aatagttttg agggatattt 1560
cataaaaacc cacagaatta ttcttgtcct caatagattt aacctttcca gaggccagaa 1620
tattctgatt ccatccttag gtagatctgg atgaacagac tccgataggt tggccactca 1680
ggcttccttt ttaaaaactc catgttgata atcacaccct gcaaatgcag tcttttaaat 1740
atgtttttgg tttattaggt aattcactca cgtgttcaaa ggtacaataa ggcttacagt 1800
ggaaaatctc tctcattcct gtttcctagt catccaggtc ccctccccag ggacaactta 1860
ggttatcagt atctgtgtat ccttccaaat atattttatt gatgcagccc aaataatatt 1920
ttctaaaaaa attgcagctt tatttacatt ccaggcatga gaatatactt tagtcacctt 1980
gtttgaactt gagtaataca ttattgtttc taatttactt tgagagatat ccttccccta 2040
aactgtaggt acaaatgtct cttagaatag gtgacttctg tagatagcta atctcatcca 2100
gtgtatctgt gtgtatatgt gtgtgcttaa ccaatttcac agagtatctg tattcttaag 2160
ttctaggaac accttcctct gtacttgttt atgccctatg tacagattct gaagtcaacc 2220
atgatataga aataataaat tcttacaacg ggctcttttg ttgtttattt tgttaatcca 2280
atgactttac ttgctttttt aatttcaaga cacatttcaa aaactctgtg ctttgttggt 2340
tggaactagg gagagttggg catatctcga aagcaacagt gatgaggaag aattgaaaag 2400
ctgaattttt gccatgcttc tggtatgttt tctattccct gtccttttag acccctttat 2460
acacacacta tcctaaatta tgcctttaag ttaagctaca aagatgaact actattagac 2520
ttgaatatgc ataacgacta ggtatgagac caccaagatc ctaaaattat tggttcaatc 2580
aaagtattta atggtatcta ctgccaatag gggaaggcaa gacaagactt cacagtctat 2640
tccatttctt ctgaataagg cagcaactaa agaccacgga gttgccaact atcactatat 2700
agatactttg cagagttggc ccagtattag acaaacaatc taatatggaa agcttgtact 2760

ttaaattgat gaggtcactc tagaaattgt taccaaattt tttcaaaaac agaacataga 2820
agagaatgaa aaaatataat tgtagtgaat gaataaaagt gtggtataaa aggggaaaaa 2880
ggacaaagaa tgaaagtaaa tcagaggcat agcaaaaagg aggagtgagt gctttgattt 2940
gcttttataa cagtaggact ggtttgggtg gcgctatcca cacactttct gctggatccc 3000
aggagataag ggtatgctct ttggctatga gtttttcatt atgtaaaact taacaacatc 3060
gtaaacttca ttattaccag acactcccaa ttcttacatc tgcaaaatta aaatagtgct 3120
tttaatgggt ggcaagagga tgtcctccaa tctcaagcag aaatggtata gtaatctcaa 3180
gggcttgtta tgtgtactta ctgtactctt cagttctttt caggagaagc acatctctgg 3240
ctgccctcag tacactaaga gaatcagacc tcagaggtgg ctgcatttta ttgttgggag 3300
taagtacttt ttaaattttg agaaatgtta gagatttccc aaaacctcac tagaagtatg 3360
aagccttgat tagaaagata aagggatttg gttccaggtc acacagcaaa ttaagtggca 3420
gaattaagtt atgaacctag acttactcct ttcaccatat tatggcattt gggggcctcc 3480
ttattgctag agtagtaaca cattacctca tatagctttt tttttttttt tttccccaga 3540
tggagtcttg ctctgtcgcc caggctggag tgtagtggct cgatctcagc tcagtagctg 3600
ggattacagg cacccaccac catgcccggc tttttttttt tttttttttt tttttttttg 3660
agacggagtc tagctctgtc gcccaggctg gagtgcagtg gcgccatctc gcctcactac 3720
aagctccacc tcccgggttc acgccattct cctgcctcag cctcccgagt agctgggact 3780
acaggcgccc gccaccacgc ccggctaatt ttttttgtat tttttagtag agacggggtt 3840
tcaccatgtt agccaggatg gtcttgatct cctgacctcg tgatccgccc gcctcggcct 3900
cccaaagtgc tgggattaca agcatgagcc accgcgcccg acctagtttt tgtattttta 3960
gtagacaggg tttcatcatg ttgaccagga tagtctcaaa ctcctgacct caggcaatcc 4020
gcctgcctcg gccttcccaa ttgctgggat tacaggcgtg agccactgca cccggcctca 4080
tacagctcta atgccacgca tcaagttctg catgtacaat ctctcatttt ggcctctcag 4140
caacccccca tgtgctaggg acagtaaatt ctattgttct cactttgcag ataagaagac 4200
tgcaattcag gaagacatat agtagtctat aagtgattca ggactggaac tcagttatcc 4260
taattccctc tcggaggctc tttccaccat gccatctgct cgtctggtgc tttccatcca 4320
tgaactctgg aaaacaagct tgagatacca aaattgaaaa gacagctcat caaaaatgaa 4380
tagttataat tctataagta caaaaatgaa atactcaaat ctcttgcccc ccctcctgct 4440
aggagcataa atgttaacct gagttctcta gctctgtaca aatccacagc aattaagact 4500
gcactgtgag ggaaaatact tttctaaaaa agaatcctga tcaagttgaa acaaaaattt 4560
taaaacatga aatggaagga acgaaactgt cgtcctccct tatttctgac ccacacttgt 4620
cgattttcat gacggagttt ctcaagccag gggcatatgg ccaaagacca tgtgaaatgc 4680
caggtgttcc aaataacaac cgacggggta ctggaaggaa agcactgcct ccccggggaa 4740
caaatggccc aatttccaga cttagttttc tgcagtctca aacccgatgt tgcaccaaga 4800
aaccacctaa tctttaggtg gtttcgttta ccgaaaaatg aggtgactgg ttcagtgcgc 4860

atatgtgaat ttaagggtaa cactgtactc acgctttgca tatttaaatt ctcaaatatt 4920
cacttaatat atgcatagac cattgtcact tcgtcagaag tcgcgtcctg cccagttgcg 4980
cgagactcca acgggccttc ttgcgacgct cgggtctggg tccgggtccg gacgtgcaac 5040
agaagccgtc agtggccccg ctggctaaaa aagggcaagc atcggaggct cgagccagcg 5100
gccgcggcgc ttcccgacag ttcctaattc ggggcgctac gccggcccca ccacctgttc 5160
ccggcagcca atggggccgc ggggggcggc cggggcggag cgcggctaca aaaggcctcg 5220
ggccccgcgc gcccgcccac cccgctccgg gcgcgctctc gggaaggctt ggaccgacgc 5280
ggcccagagg ccaggaacat tccgcgcgtg gaccagccgg gccagggcga tgctgcgggt 5340
gcggtgtctg cgcggcggga gccgcggcgc cgaggcggtg cactacatcg gatctcgggt 5400
gcgtgcgcca ccggcctcgt ctggcggccg cgtgactcac tcgatcctgc gggcggcgtg 5460
gagcactcgc tcgctccctg cccggagccg ccaccttccc tcgctttccc tagccggaat 5520
tgcacgagag tgtcccttct ggtatccctg ggaagctcag cttcaagagc cttcgcaggc 5580
tccaggagct gggcggcgtc ccccagtcag ggggaacttg cgttgcggct ccagcccttg 5640
gggtccactt gctgtctgcg cgtccgcggc ctgctggccc acggcgcagg gggcgccgcc 5700
cccctctacc ggaccttggt gcgctgcacc aagcttcccg ctcggagcaa ggggttggga 5760
cgacggaggc tcctcttagt aaaagggtgg cgttattggg gttcagtagc ctgagtgggc 5820
gtcgagttgg ggagatttct ggaaaatgcg tgtggggtag atggatgggg agaagaggca 5880
agcaaagtgg gaatcgggga cggggagcga gcgaccagat tcactccaag tataacatgg 5940
gatcccacgt ctggagagga gtcggggcga gtgcagcctg taagcagagt ttctgcgctc 6000
cccgccctcg gctgccctgg cagcagaccc tcccatgctt ctagtactgg aatcgctccg 6060
acattctggc ttcctcccag caactggttc ctgaacagct gtcattctcc gtctttcttc 6120
tgcctccacc cgagttctcc tcctgcccac ttcctaaggc ttttaacaga gcaggataga 6180
gatttggggg gttgaggttt gctcaggggt tgagggaccc tccctgctct agattttata 6240
ggccatgcat ttttaaaagg cttttgaagt ttttagatct ccttcttatt tgtttgaatt 6300
tacgcctgag ctaatcctga ccttggctag ggtactgtgg accatctgta aaatggagca 6360
gatggcagtt gctcactctc agaattgaag tgccaaagaa tgcgaagtgt atgtcaaaga 6420
tgaaaaaaat ttgatttgaa gctggttatt ctgaaagata aggtccttta caaatgttcc 6480
caacttttca tttgtaaaaa aaaataaata aaaagcctaa tattggtaac ctttgatact 6540
taagggaaaa ggaggttccg tccccagctg agactggcgg gaagttttag agcagctgtc 6600
tatggttttc tgagaggagg actgacccat ctgcaaaaca gttgcttaaa gcttgagtgc 6660
aggaattgca ttctgagata aagatggggg tgtatggtag ctggggacac ggtcttctga 6720
ggagttttaa agagttacag caattaatag ataagaagac aatttgtaga taaacaagag 6780
ggttaaaaaa attatctgta atcccactac ccacctacac actgttaacg tcttggttta 6840
tgctgttcta aaggggttta agtggcaatg ttatgctgga tttttactcc atctccactt 6900

cctcctctct gtaagtactg atagagcaat gcaatacaat aatcttttct ttgttcttgg 6960
aagcttggac gaaccttgac aggatgggtg cagcgaactt tccagagcac ccaggcagct 7020
acggcttcct cccggaactc ctgtgcagct gacgacaaag ccactgagcc tctgcccaag 7080
gactgccctg tctcttctta caacgaatgg gaccccttag aggaagtgat agtgggcaga 7140
gcagaaaacg cctgtgttcc accgttcacc atcgaggtga aggtaaagga aggctattca 7200
catgtctgaa agtgcgctcc ctcctcacct gctgctgcta ccctctgact gctttccctt 7260
cagcttactc ccagacctct caatccagtg attaagttat cttacttgac acctctgtgg 7320
tatttgacac tatttacacc aatgataatt gcttccttcc tgaagagctt cctgaagctc 7380
ttcccttgga ttctgagaga gcttttctgg gaataatagt tctttcactg tcctcagatc 7440
ttcttccgtc acagcctgta atggcaaggt tgaacagaat attactttca gcccccttct 7500
cttagccatt ctcacagcta ccgctcccac ctcctctctg gtccggatct ctgtattgaa 7560
ctccttccag atcaacgagt taaactcctt acgggatatt gccacaggac ttcaaacttg 7620
gcacatctgg agttcagttg ccagccacct gctcaagtgg atccctcctc attcctgcct 7680
tttaattatt ggcattccta tttaatcaag ctaaaacaca aaagaatcat ctttgatttc 7740
tccttatgtc acgtgatgta acaaggcttt tgtcaaaaat gaatcttgcc tcattcctct 7800
gctttgcctc tccctaaagg ttgggagctc atctgctgcc tggactattg cagttaactc 7860
ctaactggtc actcctactc actcttctca ccaaatcacc tggcagctgc aatccaagtg 7920
attttttaaa actcagatgt gatcatgtta ctcatcctgc ttcttttcct cctgtacttt 7980
gctaggcgac ccccactacc ccaccacccc ccgcattgtt taggataaag tcttcatggt 8040
attcaagatc cttcacaatc tgactcttgt ctgcatttcc aggattcccc attccagtta 8100
ttgactcatc tgttagtaca gcagattagc aggtgttcat ttctggtcaa gtccctctat 8160
cctctaatat tccaccctgg ttttcagcag actttataag acaaccatat aaggcagcta 8220
agctagacta ctcactgttt cttcacaagc cctgcatctt tgcccctacc taagtctctt 8280
ccatccatct tcctgtgccc tttagtctgt agtttaaacc taccaccacc tagcttcaat 8340
gacacctttc acaaagtctt ccaggatttc cctggcaaca agtaatatct ctctttttaa 8400
aaactcctag tggtggccag gcgcggtggc tcacacctgt aatcccaaca ctttgggagg 8460
ctgaggcggg tggatcacga ggtcaggaga tcgagaccat cctggctaac acggtgaaac 8520
cccgtctcca ctaaaaatac aaaaattagc caggcgtggt ggcgggtgcc tgtagtccca 8580
gctacttggg aggctgaggc aggagaatgg catgaaccca ggaggcagag gttgccgtga 8640
gctgagatgg cgccattaca ctccagcctg agcaacagag tgagactcca tctcaaaaac 8700
aaacaaacaa acaaaaactc ctagtggttt ttatctgtaa cgttcttata tccagggcct 8760
ctatgggctg gtgttcaggt tctatactgc ataaggattc catatctaag ggaccacttt 8820
ccacattgta gacacagcct agactcctat taataatggg ttacaaatgt atctttagtg 8880
tccttaaatt tttcctgaat atttacactt ttatttgtaa agtggattga ggaaagaagt 8940
tctgtaacag cattgtcgtg gtcttcagaa atattgccta ccatgtacca gggctctaga 9000

ggtattaata taaaagatct tcctttctat ttgaagagat ttgatgttca catagaaacg 9060
aagaccaagt atcttattcc agtcgactga caaggcagtt gaagtgccag gaaagaggaa 9120
tgaccaaggg ctggccagtt aagaagtcct cttgaagaaa atgactcctg agttagactt 9180
ggaagacagg gaaatagaaa aagtaataac ttctaattgt tctgaaacta tctttggttc 9240
tgatatttgg aatttgatga gaagattaat ttatacacca tccacatgtt tttaaaaggc 9300
atagtagtct ttttaagtct tttcttgaaa agtcccatcc cctaaccatt ttaattgggt 9360
gaaattaaaa tttaaatcca agcaataatt tatccttcta cttgtcttta aaatgataga 9420
ctttcatatc atgcgatttt acagaatcag aattctcaga ggtaattttg agttctttgt 9480
cacttgattt atgactttag actttgtcat ttcatggact tgtggaaaaa gtaccatgct 9540
gaagtcagga gtctgggatt aattaggtat ttgataaaaa cttgatagct agtctcggtt 9600
acttagctat ttgaccttgt acaagattcc acaaatttct gggccacagt aaaatgaaga 9660
tattaagagg tttgctagtt tgctatctac ctaacaggat taaggattaa tctaaagata 9720
aaaggttaca tgttaagtat tttaaaggac aacacaacaa cttattatag ttgtatggat 9780
ccagtttgcc aattacattc caaaggtact ttgtcggtgt aatagttgta gatatcagta 9840
acattagcag tattgatttc attcgtctgg ttctttttgt ttgtttattc ttctttctct 9900
ttctctcttt gattctagac aggaaataat ggttatttct gtttcatgcc atgtgctaag 9960
cctggttttg agggagaaga aagtaaatct aagacatgca tggatcctag caagcaaggc 10020
catgcctcgt agtagagaag aatgacacat atattaatca gtttgggaac aataaaagaa 10080
aaaataatgt agtaaactgt ccataggcaa tcagagcaga agatgttgga agatataagt 10140
actagagaaa ttttaaggaa aggaatgtgg aacataattg ttgggaagca taatttttta 10200
aagctctgtg actttttgtt ctgcaaaata tagtaaatat ctgctatcaa atatatatat 10260
atatagatat atattttttt aagatggaat ctcgctgtct cgcccaggct ggagtgcagt 10320
ggcgtgagct cggctcgctg caagctccgc ctcctgggtt catgccattc tcccacctca 10380
gcctcccaag tagctgggac tacaggtgtc ccccaccagg cctggctaat ttgttttttt 10440
tgtattttta gtagagacgg ggtttcaccg tgttagccag gatggtctcg atctcctgac 10500
ctcgtgatcc acacgccttg gcctcccaaa gtgctgggat tgcaggggtg agccaccgtg 10560
cccagcctca aatatgtttt ttgttttgct tttgtctttt aaaaataaag cccttgataa 10620
aggacctttt ataaaataca agattaatca agattagtgt aatataagct tacagctgga 10680
taaatacatt tctgttgtca tttcttagcc ttccaaagac agtatttaaa aattcacttg 10740
gcaaaccttt gtaaagggtt ttaggctttg aaagaatata aaggttcaag gaatgcagac 10800
atacctttgc aacagattat taaaagcatc atatgctaag cgtctaatat agtgttcatc 10860
atcttgcaaa caacttggtg aatgattgct ggctcattta gcagggttat aaaggaagtg 10920
taaacaattt ctagctatct cttgtaaaac gtattgtgcc ctgatcattt gtactttgcc 10980
caacctttgc cagattataa actctacaag aaaaaatcac atttgttcaa ataacctttt 11040

tctcccagga aggtttaatt tatcaaggca agcttcaccc tgcacaaata gtcacggagg 11100
atactttgga atgagtctgg caagacagca taactctgca tctgctgtaa cccagtgtta 11160
aattttcaaa ggcttcttgt ccctagtgga ttgtaggaga tgtgcctggc atgagaagga 11220
ggaagtggtg aatggatgag tagtaccagc atgtatctga gaggaatata aagagtcatt 11280
attatcctct ttctggttgc tgttgcagaa attcctagga gtagaaaaca gtggagtgct 11340
tttattttgt tttttgaaag ggtagcttgg tctttaaaaa tgtttaatga tgataacatt 11400
tttcattagt ccagaaattc agcctgctgt ttgcctatag accttagtaa aagagctaat 11460
taatgattca gaccccaggc gtcagcaaac ttcatctttc taccagataa taagtaattt 11520
aggctttgca gaccatcggt ctctgttgca actactcaac tctcagctgt gccattgtag 11580
tgcaaaagca gccatagaca atacgtaaac aaatgaatgt gcctgcgttc ctgtaaaact 11640
ttatttacaa aaacaagcag caggccaaat ttgcctatgg accttttttt accagcctct 11700
gtttaagact ttcactggca tcattcagtt tttctaaaga cctgtttctt ggcttattcc 11760
ttattcacca agctaggttc cagcgtaact gatgtgaacc tggatattga actaaccaga 11820
gatgggtcca ctttcatata tttgatattt gtcagactga gataaaaatg gaagtcgaga 11880
cacaaaatct agaaaagata ttgtttttga cattaaagag ctaaacatct agacatttga 11940
tcagtcttga tgagtaaaag ccattgggac aatttgaact tgttaagcaa ttccagtctt 12000
aaaagttttt gcacatctag ttagaagtgt tcaaaccagt ataacatggc caagtcccat 12060
gatgtattag ggacactctt tgagtaacag tcagagaact tgagttctag tcctgcttgt 12120
ccagatagct agcaagcatg tgacctgcca gagcctcttt ccttacatgt gaaataagca 12180
tatgagaact tgccctgttt ctttgatagg attgctggga aaccaaatga gaatatccag 12240
ctaaagtaga cacactgtga cacacttgag cccattacca agttttacac ctttctagta 12300
cttattgcta tctgaaatta tctgatttat ggtggagact gtcatatctg acatgatcag 12360
gactagtagt ttgtcagttc atcaattata taaataaaca tttttaaaat acctgcttta 12420
accagtttta aacaaaaatt taaattaaaa cacttaaaac atttttaaac ttaaaatata 12480
tcaatgtaca ttgctttgct aatcttttga tgaaggttca aggctttttc ttagagtctg 12540
gattctctga ttggcgttct gcttcattta gcttgaaaaa tgggaagatg catcacgtac 12600
atttctcaat gtatttatga ttgagaaatt gactaagaac ttagacattt ggaaaacaat 12660
ctgaatgcag aatctgtcta gatttttaca tttttgaaaa ccaatatttt ggccgggcat 12720
ggtggctcac acctataatc ccagcccttt ggggggccga cgcgggtgga tcacgaggtc 12780
aggagattga gaccagcctg gccaacatgg tgaaaccctg tctctactaa aaatacaaaa 12840
aattagccgg gcgtggtggc atgcgcctgt aatcccagtt actcagggag ttgaggcagg 12900
agaatcattt gaacctggtg ggaggcggag gttgcagtga gctgagatgg tgccattgca 12960
ctcctgtctg ggcaacagag tgagactcca tctcaaaaac aaaaacaaac aaacaacaat 13020
atttaacagc tgttgcacct accctattca acaataattt ttagcaaact gattgagcca 13080
ttttaaagca ttcaaatgtt ttctctttag ttgtcttcta aaatacacat agccctggaa 13140

actttctaaa ttttttttca gttattaaaa atttgtgaat ttgcttatca tatcacttcc 13200
ttgtatgaca aaaagacttg aagagatgtg agagctgaat ggattagaaa aaggagaggt 13260
gtggttggta tgaggcatat tgacagggaa ttaccaaagg cagatttgca ctgtcacaca 13320
attttggcaa gtgttaattc tgttgaatgg gaaaatcaat aatgtttttt tttttctttt 13380
tctttttctt tttctttttt tttttttgag acagagtctc gctttgtcac ccaggctgga 13440
gtgcagtggc acgatctcag ctcactgcaa cctctgcctc ctgggttcaa gcaattctcc 13500
tgccttggcc tctgaagtag ctgtgactac aggcacgtgc caccatgcct ggctaatttt 13560
tgaattttta gtagaaatgg ggtttcccca tgttggccag gctggtcttg aactcctgac 13620
atcaagctat ctgcccgcct cagcctccca aagtgctggg gttacaggct gagcaaccat 13680
gcctggccaa taatggggtt ttaagagaaa taaagcatat ttgaaagtat gctcctgatc 13740
ttaacggggc aaaatatctt tttttttccc ttcacaaaat atcttgaggt gctattttct 13800
agacagaatt ctgagatgca tggatagccc atggttttgg cttagtataa gtgcttttat 13860
tctcttatgc tagtttgtgt gttctcttgt ttcattttat tgaggctctc taatagaagt 13920
gctatgtcct gttttgtctg caaagcatgg caaaaaaact ggccaagagg attccaagtc 13980
tgtctgaagg taaacatgct gttagtggtt ggccatgaga tctcaggcag gaggagcaaa 14040
aggcctcaaa atatcaagat gttttagagt tcttcattca acagatactg agtgtctgcc 14100
cagagggctt atgtgctcaa tgctagggat cagcactgaa catggagtgg ttggagtctc 14160
aattccaata ccttatacaa agagctgttt actgcctatg aacctatcat gagctgtatt 14220
tttctcctgt aagtattgtg tttatttgct tctacttcca ggccaacaca tatgaaaagt 14280
actggccatt ttaccagaag caaggagggc attattttcc caaagatcat ttgaaaaagg 14340
ctgttgctga aattgaagaa atgtgcaata ttttaaaaac ggaaggagtg acagtaagga 14400
ggcctgaccc cattgactgg tcattgaagt ataaaactcc tgattttgag tctacgggta 14460
agtgatgtca gataactaaa ctgccagtgt ctgaattttc aatttctttc ttaatgtgta 14520
aggggaaagc tccaaaaaat ttcttggaga gtcctttgct ttgctagttc ttgaaaactt 14580
taaagattta tttaggaatg gtttggtcca tgttgatatg attaagatgt atacattcta 14640
aactccattc catctcgtgg aatctccttg aaatgacaag ttttcctaaa ctttatagcc 14700
atttccttac ataataacag tggattcgtt taattttcct caagaacaga aattaaaagg 14760
ggaatagaaa catttccatt ttatttttaa ctgacattgc tgcatgactg ttcttgtgcc 14820
ttaggtcact gttctaattt ctagttttag attgatcctt ggctcaattt ttatcttgtg 14880
ataccgatag tcctttaaga gctgtgtttc tgtatagcaa gaaaggagct tacaaatggt 14940
gaaagcttgg tggttcaaga tctctttgta caaggacttt attccttcac ttttttttct 15000
tttctttttt ttctgggctt cccccccacc acacaattca tgttaacaat ctagtatgtg 15060
ttattcgaat tttttgctat tttcatatat atatacacac atgtatttct agcaattttt 15120
ttaaccaaaa aaagaggtaa tattatatag acattttttg tttgtttgtt tgagacagag 15180

tctcactctg tcgcccaggc tggagtgtgg tggcacaatc tcggctcact gcagcctttg 15240
cctcttggtt caagtgattc tcctgcctca gtctcccgag tagctaggat tacaggcacg 15300
tgccaccatg cctggctaat tgttttcgta ttcttttagt agagatgggg cttgaccatg 15360
ttggccaggc tgctcttgaa gaggatcaag tgatccgccc gtcttggcct cccaaagtgg 15420
tgggattaca ggcatgagcc actgtgcctg gccctatata gacctttttt gtcttggaaa 15480
ttaacactac tgaagttgtc atggaatttt ttggtctgac aggtttatac agtgcaatgc 15540
ctcgagacat cctgatagtt gtgggcaatg agattatcga ggctcccatg gcatggcgtt 15600
cacgcttctt tgagtaccga gcgtacaggt caattatcaa agactacttc caccgtggcg 15660
ccaagtggac aacagctcct aagcccacaa tggctgatga gctttataac caggtgagtc 15720
cctaattata ggctagctta ttactaactt ttctaaatga tccacaaaga gtaagataga 15780
gattctctga taccttgtat atattatgca ttttagaaac cgaaaacatc atcttatttt 15840
taaaaagtga ttattttatg atgaatatga taagagtaaa tgatcctatg ataataataa 15900
taatggctaa cactttttgg tgcttacctt gttctgggtc ctgggttaaa ccctttattt 15960
acatcctcaa tcctgtttgt ttttcacaac aaccctaatc atactgtagt atcattccat 16020
tagatgggga aaaacttgcc tacaggtgta ccactgttca ttagtggaac cagaaaagtc 16080
actagactga agttcacttt cttcttctag aaaaagaacc tgatctacag ttcaaattca 16140
cagtaaaatg aaatgtatgt cccttattaa atcagttacc atttgatctt ttatagaaaa 16200
aaatttaaaa atcaaaatag cttctgggtt taaagacaga aataaataat gaattagttt 16260
tgccactccc aatcaaattt ggcaggggaa acatggcatc tcgttttatg gtatgagtta 16320
agctcgggac caaattcctt ttctaaaaga acagaatgac ttcatcctgt ttactttccg 16380
tacggttcat ggaatattct cagaatcagt tactgcctct gtatcacttt tttttttttt 16440
ttgagatgga gtcttgctct gtcacccaga ctggagtgca gtggcgtgat ctcggctcac 16500
tgcagcctct gcctcccggg ttccagcaat tctcctgcct cagcctcctg ggtagctggg 16560
attacaggtg catgccacca tgcctgacga atttttgtat ttttagtaga aacggggttt 16620
caccatgttg gccaggctgg tctcgaactc ctgacctcag gtgatccacc tgccttggcc 16680
tcccaaagtg ctgggattac aggcatgagc catctcacct ggtgtgtacc atttttttct 16740
aagtacatag tgccccttca tcctcagggg atatgttcca gactctcagt ggatgctgat 16800
actgaactct atatattttg ttttttcctg tacatatata catacctatg ataaagttta 16860
atttacaaat taggcacagt aagagcctaa caacaataat gaataataaa atggaacaat 16920
tatataaata tactttacta aaagttaggt gaatgtggtc tctctgtttc tctcaaaata 16980
tcttaatgtt ttgggacgac agttgactga aggtaactga aaccatggaa agtgaaatca 17040
tgggtaaagg aggactgttg tatcaggaaa ttactataat gctagtttct tgttttattg 17100
ttgttttggt ttatttatca tggacccgct tctttcccag gcatgtttta gtctagaact 17160
gagtagtaat attttgggaa atgatgttaa actaattttc ttagtactgt atgccttatg 17220
aataattttc tcatgatcca gtgcattgtg tgtgttcatt cttgttccaa ttaggattat 17280

cccatccact ctgtagaaga cagacacaaa ttggctgctc agggaaaatt tgtgacaact 17340
gagtttgagc catgctttga tgctgctgac ttcattcgag ctggaagaga tatttttgca 17400
cagagaagcc aggtgaacgc aggctgcctc tgaaatttca cactatatta ttgtattctt 17460
tacttccaaa ttaaaatatt ttcatcctaa atttttttca ccttgaatat ttttagtttt 17520
ctagacatag aaccaaatat ttatcgatcg tatgtctttc ttttgttttc aggttacaaa 17580
ctacctaggc attgaatgga tgcgtaggca tcttgctcca gactacagag tgcatatcat 17640
ctcctttaaa gatcccaatc ccatgcatat tgatgctacc ttcaacatca ttggacctgg 17700
tattgtgctt tccaaccctg accgaccatg tcaccaggta agagatttct tgggactctg 17760
gaaaagcaac agaatctagt aactaaaata attgccactt ttattttaag cacttccatg 17820
gttcctaatg gttctaaatg acagattcag cagtactcaa cattattatg atcaaacatt 17880
ggattcctta tggcttatta caagatgtcc accatgcaga aatatacata gttgacacaa 17940
taattagtat gggatcatca gactttggcc acttgctgtt gtcagagcct caagtttgtg 18000
catatgtgtg tatttttatc aaacgatggt gttataactt gcaacagtgg ttccacagca 18060
gcgaattttg gacaagatca tccttgcatg gccagggcaa tgccactggg cagtggttgt 18120
attccctcta cagtggggta gcctcccttt ggccaaccct gctctgtagg gaaaagtgaa 18180
gccactgcac tgcagctggt ctatcccctt cacctgcagc ctcctacttt ctgtccatag 18240
cctcctactt ccagttaggt gatgagtgat tttctgttac ttgcaaggct gcagtacaac 18300
ttttaaaaac cactgttata ctgtgtcact gttcatttct gtttttattt tttaaacccg 18360
atgaccatga ctacttattc atttcatttt attatcattt attcgtatag taagtgggta 18420
tgtgaacaca gggacggcta agttgtaaaa ggtcactaag gacaatatgg ttctttgaga 18480
atgcatagct aacaactgtc tttataccat ggtcctagat acaaaatgat gggcaagaag 18540
tagaaggact gtgggtttga tgtagtcaca tttttgtttt cttgtaacta aataggaaat 18600
ttttcaattt ttaaaatgag aatttcttaa ggacagtatc tttggttttt taagtctctg 18660
aaacctgaga aaagttaaga ctatctttaa aagctgtaac atttctgtta ctttttaact 18720
ttaatctggg gattttatgt acatttttaa ttaggattca gacaaaatca cccaaatgtt 18780
tagaaatttt aatttctttc ttttggaagc tgagaagaca gcttctcaaa gagaattatt 18840
actgataagg gctattagag actaataata ataatagtat atgtagattt aacggttttg 18900
gggggttttt ttggtcttac agattgatct tttcaagaaa gcaggatgga ctatcattac 18960
tcctccaaca ccaatcatcc cagacggtat gtttactttc atacactgtt agactaaaat 19020
aattacttta gtgaccaagg atctttctcc tatagagagc agcccaaatg ttagcagctt 19080
agagaatgtg tttcctcagt aaggtgtgaa gtggtgcttg ggactgtgag catcagaaca 19140
aggctgggac ttgtgtttgc aactttttca tccatagaaa atgaagtaag gagaagacag 19200
aataaaacaa catcacgtaa agcaagatat gacttttttt tttttttgga gacagtgtct 19260
tgctttgtca cccaggcttg agtgcagttg tgcagtcaca gctcactgca gcctcaagac 19320

tcctgggctc aagtgatctt tccactccag cttcctaagt agctgggact acaaatgcag 19380
gccactatgc ccagcaagtt attttttttt atttgtagag atgatgtctt gctgtgttcc 19440
ccagtgaggc accatctctg ggcaatagtc tcaaactcct aggctcaagg aatcctcctg 19500
cctcagcctc ccaaagtgct aggattatac catgcccagc caagatatga cttttaaaag 19560
aaaacagggt gaaattcaaa tgtataatga gtacagattg attcatacca tttatagata 19620
aattgtttta agattaaaat aaatgttcac atggaaagtg aactactgaa agaactgagc 19680
tgtcacttta atattcttca cttgtaaatc cttgctttaa acaaaaatga tagcggacat 19740
ggtttaacag ttgttgctgt ttttagatca tccactctgg atgtcatcca aatggctttc 19800
catgaatgtc ttaatgctag atgaaaaacg tgttatggtg gatgccaatg aagttccaat 19860
tcaaaagatg tttgaaaagc tgggtatgta caacaaatga aacatgttgt catacatgaa 19920
aacgttaaat gagaagaact aaagaaatga catgctaggt ttgataaatg gtttgtctaa 19980
ttcaattttg attcctttta gaggcaccaa gttattattt cacagaaagt cagtattttt 20040
attttcaaca cacgtagccc caacaagttg gagatgcatc ctgagctcct gagacatccc 20100
cttaacaata agtttataaa tcttatatga cttagccaac gttttggtgc ccatgtgcag 20160
aaaaccaccc ctttgcaggt gagcagatag cttgttcagc ccttcttcaa ataagaaatc 20220
attgtgggtc ctttaaaagt gtcctgtcat cacatatttc aaaggactca aaagggtacg 20280
gatgcagaag tgacttttat gtgtgataag catagttaac aaagtagcga aatgcaagaa 20340
agtgtgtcag taaatcattc agttttgaac tgaagagcag gaccaggttt tacttaagta 20400
gtttacttat cctgatattc aaaatttaga ccaatgattc tagtattatt gagaaaaatg 20460
ttgaagggaa aggattttca tatgagctat tataaagggg tgtgtgtgtg tattttgaga 20520
cagagctgtg tcgcccaggc tgtagtgtag tggcgtgacc tcggctcact gcaacctaca 20580
ctttctgggt tcaagcgatt ctcctgccgc agcctcctga gtagctggga ttacaagcat 20640
acaccaccat gcctggctaa ttttgtattt ttagtagaga ggggcttggc caggctggtc 20700
tcgaactcct gacctcaagt gatccatcca cctatgcctc ccatagcatt gggattatgg 20760
gtgtgagcta ccatgcgcga ctaggaaagt ctttctgaat ggctcatgcc accttcctct 20820
ccctggctat accaatggac cctttagtga ctttgtagcc tatcctcctc tcaaaataat 20880
tagctccttg aggaagaata atagattttc tttttaaaac cccactatat ctaacacaac 20940
agattcttcc taaataccat tggttaaata ggccatgcac atcatagcct tgtcattaca 21000
gatggtacca gattaaactg attgttggat ttgctttggt acctgtataa ccatacaaac 21060
ccagatatta aaacaccagc tctgttccat tgtttcctta tggtggcctg gtgaagttaa 21120
tctgtatcag ataaactctc cttatatgtg aatatgtaaa gtcagctttt taggtcagct 21180
aaattttgct ttgcctacta aattaacatg gtaaaattag aaacagtctg acaaagtagt 21240
taatgtataa ttctaaaagg ccttgagatt ttagtacttg accattttga acatttggta 21300
ggtacatctg ctcagtctat tgacttgtga atagtcaata gaacaaaact tcagagttct 21360
ctgttgactt tttggagaag attttatatt agcagatgaa atatagttgg tgaaaataaa 21420

attggaagag aaaatactat tctgggttgg gtatccttaa ggattagaac cacaggactc 21480
ctccaagtct gtaggcctac tttctctatg agaatcgtca tgttagtcca tctaactgac 21540
ctcatctctc tttcaatgca ggtatcacta ccattaaagt taacattcgt aatgccaatt 21600
ccctgggagg aggcttccat tgctggacct gcgatgtccg gcgccgaggc accttacagt 21660
cctacttgga ctgaacaggc ctgatggagc ttgtggctgg cctcagatac acctaagaag 21720
cttaggggca aggttcattc tcctgcttta aaaagtgcat gaactgtagt gctttaaaca 21780
atcatctcct taacaggggt cgtaagcctg gtttgcttct attacttttc tttgacataa 21840
agaaaataac ttctgctagg tattactctc tactcctaaa gttatttact atttggcttc 21900
aagtataaaa ttttggtgaa tgtgtaccaa gaaaaaatta gtcacctgag taacttggcc 21960
actaataatt aaccatctac ctctgttttt aatttctttt ccaaaaggca gcttgaaatg 22020
ttggtcctaa tcttaatttt ttttcctctt ctatagactt gagaatgttt ttctctaaat 22080
gagagaaaga cttagaatgt acacagatcc aaaatagaat cagattatct ctttttttct 22140
aaaggagaga aagacttaga acatacacag atcctaagta gaaccaggta attgtctctt 22200
tttctaataa ggaatttggg taatttttaa ttttttgttt tttaaaaaat aacctagact 22260
atgcaaaaca tcaaagtgaa ttttccatga atgtttttaa tattctcatc tcaacattgt 22320
gatatatgct actaaaaacc ttttcatata catcttacct catttcaagt gaattatttt 22380
aatctttttc tctctttcca aaaatttagg aatgtttagt gtaattggat ttcgctatca 22440
gttcccatcc ttaagttttg atattcaata tctgatagat acactgcatc tttggtcatc 22500
taagatttgt ttacaaatgt gcaaattatt tagagcatag actttataag cattaaaaaa 22560
aactaatgga ggtaaaacct aaatgcgatg tgaaataatt ttagtgttga taccgtatgt 22620
gtatttttat tctaataaac ttttgtgttc cagattgaat ttggcattgg tctagtcatt 22680
ttagtataag taaattatag gaatgtggct cttggtacag aattctaatt ctctaaaata 22740
atcaacatat tcatcacaaa agtaaaccta atacattcag aaatgtttag caaagtcaga 22800
tttttgtttg ttaattaatt cactcattca aaaatatctg aatatcttct gagtataagg 22860
agtaatccta ggccccggaa gggaagagtc tctgttctta agttgctgca tgtttttttc 22920
ttgaaaaatc tgcatagaat gtgtactttg aaatgaatca caggtttcaa ctgtaccctt 22980
actaacgcct tgaactcctt tgcatcctgg tgttcctttt acatccatta cagaaacacc 23040
tgttagtcct acacgtggac tgaattctgc ttgagaaatc tatccagttg tttggattgg 23100
tgcacattag ggtatatatt tgcaggccta aatactaccc aacaattctg ctgcacagtt 23160
ctttgcgtcc tcctcagtag ctattccaaa ccttcactac attttcttag ctgatcatat 23220
tgtttcattt ttcacaaagg agagagagac catcaggcat tagttccttc agtttacgat 23280
cccctataga tttatacaca tttgcatatt ctggttaagt ttctttctcc tttcctttac 23340
agtaagtagt tctcccttat cctcagggaa tatgttccaa gacccccggt gaatgcctga 23400
aactatgggt agtaccaaac cctatatata ctatgccttt tcttatacat gcagacctat 23460

gataaagttt aatgaagtta atctggtgca acacatggct gtcagtcggg aacatgtttt 23520

ctcttcatgt cttgcaaaca aaaatgtaat gccttttcca ccttaactaa gcacttatca 23580

atgctctgtg accataactt ttgccatgtg tagtgcgaca gcaaaactag catgcatttc 23640

tttttccttt atcacaactt cacagatagg agattcgttc ttaccatgga tctcagcaac 23700

ctcaacatac aactttttct tttcttgttg agaacttcca cctttccctt aaagaaagcc 23760

acttaggctt tactttggta tgtccgaatt gccagcatta agacttgcac ttgagtcatt 23820

attaagtaaa ataaggttac ttgaatacaa ggactgtggt ctgataacga ggtggctact 23880

aaatgactga agggcgggtg gtatatacag tgtgatacgc tggaagcggg aggattcatg 23940

tcccaggtgg agggagtgga gtgggccagt gcaagatttc atcacgcagc tcagaacagc 24000

gtgcaattta aaacttacga attatttatt tctgcatttt tctatttaat atttttggac 24060

catggttgac cactggtaac tgaaactgct gaaagtgaaa ctttggatga agggactact 24120

gtgctaccta agcttccaga gaaaaaaatc aggccttcat ctcattgcat gcatttgctt 24180

ctcaaccatg tataaagggt cttctctcgc tgctacagaa aaagaggtga aacttctcac 24240

caactctaat ccacacaacc gtcctttgat cctaatcttt ctcttttttc agaatttgct 24300

acatcaatta tcatctttct tgtaactcca gcaccaccac cgctctgtgt gtgtgggagg 24360

gagctttccc cctcagaaaa tagtcatgct caaaacaatc cacagtgcgg tctacatccc 24420

tgatccgtga tcaaggcctt atcatgacaa aatgatcact cctagtttga gagctaccac 24480

ttgctttgca atatcatata atcaaataga taatcaaaat gtacatacag attccacaca 24540

tttttaataa ccagttggtt tggacttata cagatagctc aaccaaccaa aaattctatc 24600

ataagctttc tcatatattt gaactaactt tacaacaatt catttgccta cctgatctg 24659

<210> 17
<211> 2602
<212> DNA
<213> Homo sapiens

<400> 17
ttgcgacgct cgggtctggg tccgggtccg gacgtgcaac agaagccgtc agtggccccg 60

ctggctaaaa aagggcaagc atcggaggct cgagccagcg gccgcggcgc ttcccgacag 120

ttcctaattc ggggcgctac gccggcccca ccacctgttc ccggcagcca atggggccgc 180

ggggggcggc cggggcggag cgcggctaca aaaggcctcg ggccccgcgc gcccgcccac 240

cccgctccgg gcgcgctctc gggaaggctt ggaccgacgc ggcccagagg ccaggaacat 300

tccgcgcgtg gaccagccgg gccagggcga tgctgcgggt gcggtgtctg cgcggcggga 360

gccgcggcgc cgaggcggtg cactacatcg gatctcggct tggacgaacc ttgacaggat 420

gggtgcagcg aactttccag agcacccagg cagctacggc ttcctcccgg aactcctgtg 480

cagctgacga caaagccact gagcctctgc ccaaggactg ccctgtctct tcttacaacg 540

aatgggaccc cttagaggaa gtgatagtgg gcagagcaga aaacgcctgt gttccaccgt 600

tcaccatcga ggtgaaggcc aacacatatg aaaagtactg gccattttac cagaagcaag 660

gagggcatta ttttcccaaa gatcatttga aaaaggctgt tgctgaaatt gaagaaatgt 720
gcaatatttt aaaaacggaa ggagtgacag taaggaggcc tgaccccatt gactggtcat 780
tgaagtataa aactcctgat tttgagtcta cgggtttata cagtgcaatg cctcgagaca 840
tcctgatagt tgtgggcaat gagattatcg aggctcccat ggcatggcgt tcacgcttct 900
ttgagtaccg agcgtacagg tcaattatca aagactactt ccaccgtggc gccaagtgga 960
caacagctcc taagcccaca atggctgatg agctttataa ccaggattat cccatccact 1020
ctgtagaaga cagacacaaa ttggctgctc agggaaaatt tgtgacaact gagtttgagc 1080
catgctttga tgctgctgac ttcattcgag ctggaagaga tatttttgca cagagaagcc 1140
aggttacaaa ctacctaggc attgaatgga tgcgtaggca tcttgctcca gactacagag 1200
tgcatatcat ctcctttaaa gatcccaatc ccatgcatat tgatgctacc ttcaacatca 1260
ttggacctgg tattgtgctt tccaaccctg accgaccatg tcaccagatt gatcttttca 1320
agaaagcagg atggactatc attactcctc caacaccaat catcccagac gatcatccac 1380
tctggatgtc atccaaatgg ctttccatga atgtcttaat gctagatgaa aaacgtgtta 1440
tggtggatgc caatgaagtt ccaattcaaa agatgtttga aaagctgggt atcactacca 1500
ttaaagttaa cattcgtaat gccaattccc tgggaggagg cttccattgc tggacctgcg 1560
atgtccggcg ccgaggcacc ttacagtcct acttggactg aacaggcctg atggagcttg 1620
tggctggcct cagatacacc taagaagctt aggggcaagg ttcattctcc tgctttaaaa 1680
agtgcatgaa ctgtagtgct ttaaacaatc atctccttaa caggggtcgt aagcctggtt 1740
tgcttctatt acttttcttt gacataaaga aaataacttc tgctaggtat tactctctac 1800
tcctaaagtt atttactatt tggcttcaag tataaaattt tggtgaatgt gtaccaagaa 1860
aaaattagtc acctgagtaa cttggccact aataattaac catctacctc tgtttttaat 1920
tttctttcca aaaggcagct tgaaatgttg gtcctaatct taattttttt tcctcttcta 1980
tagacttgag aatgtttttc tctaaatgag agaaagactt agaatgtaca cagatccaaa 2040
atagaatcag attatctctt tttttctaaa ggagagaaag acttagaaca tacacagatc 2100
ctaagtagaa ccaggtaatt gtctcttttt ctaataagga atttgggtaa tttttaattt 2160
tttgtttttt aaaaaataac ctagactatg caaaacatca aagtgaattt tccatgaatg 2220
tttttaatat tctcatctca acattgtgat atatgctact aaaaaccttt tcatatacat 2280
cttacctcat ttcaagtgaa ttattttaat ctttttctct ctttccaaaa atttaggaat 2340
gtttagtgta attggatttc gctatcagtt cccatcctta agttttgata ttcaatatct 2400
gatagataca ctgcatcttt ggtcatctaa gatttgttta caaatgtgca aattatttag 2460
agcatagact ttataagcat taaaaaaaac taatggaggt aaaacctaaa tgcgatgtga 2520
aataatttta gtgttgatac cgtatgtgta tttttattct aataaacttt tgtgttccag 2580
attgaaaaaa aaaaaaaaaa aa 2602

<210> 18
<211> 423

<212>  PRT
<213>  Homo sapiens

<400>  18

## Description

### METHODS FOR DETECTING THE MORTALITY RISK

The present invention generally relates to methods for determining the mortality risk in a subject. In one aspect, the invention relates to a method for predicting the all-cause mortality risk of a test subject based on the detection of pathologically decreased expression levels of the gene encoding the L-arginine:glycine amidinotransferase (AGAT) and/or pathologically decreased activity levels of the AGAT enzyme. In a further aspect, the invention relates to a method for predicting the all-cause mortality risk of a test subject based on the detection of genetic alterations which are associated with decreased expression levels and/or activity levels. Finally, the invention also relates to methods of screening for compounds which are effective in increasing the expression of the AGAT gene and/or the activity of the AGAT enzyme.

### BACKGROUND OF THE INVENTION

Acute ischemic stroke is one of the major public health problems causing high rates of mortality and morbidity in cardiovascular patients. Consequently, considerable effort has been put into the evaluation of factors which may provide predictive information on morbidity and mortality after the acute event. Besides the traditional risk factors (advanced age, hypertension, atrial fibrillation, diabetes mellitus, and smoking), factors involved in hemostasis were found to predict mortality after acute stroke, including von Willebrand Factor (vWF) and β-thromboglobulin (βTG) (Carter et al. (2007), Stroke, 38(6): 1873-80) which reflect the activation of endothelial cells and platelets. Besides these well known risks, identification of vulnerable patients is still a challenge for modem medicine. Thus, also the identification of biomarkers which are related to stroke outcome is highly desirable.

Recent data has suggested that low homoarginine plasma concentrations may be associated with cardiovascular and all-cause mortality in patients subjected to coronary angiography or hemodialysis. Homoarginine is a cationic, non-proteinogenic amino acid that was described first in sweat peas (Bell (1962), Nature, 17, 193:1078-9). Homoarginine differs from L-arginine by a single methylene group and appears to be involved in the nitric oxide (NO) pathway. See, e.g. Kakoki et al. (2006), Am J Physiol Renal Physiol., 291(2): F297-304; Moali et al. (1998) Biochemistry, 21, 37(29): 10453-60). The molecular regulation of the homoarginine metabolism has so far not been elucidated. Formation of homoarginine by enzyme catalysis similar to urea cycle reactions has been proposed as putative metabolic pathway in humans (März et al., (2010), Circulation, 122:967-75).

Against this background, it has been found by the present inventors that the homoarginine concentration in the plasma of a subject is related to survival of said subject after acute ischemic stroke. Further, it was found in a genome-wide association study that the formation of homoarginine is directly associated with the activity of the enzyme L-arginine:glycine amidinotransferase (AGAT).

As described herein below, the inventors found that the catalytic activity of the AGAT enzyme is required for the synthesis of homoarginine in mammals. This insight provides the possibility of measuring the homoarginine concentration in a subject or, alternatively, the expression level of the AGAT gene and/or the activity of the AGAT enzyme to assess the individual mortality risk of said subject. Based on the insights provided by the present invention, diagnostic tests can be developed which are useful for detecting alterations in the genome of a test subject that are associated with a decreased expression of the AGAT gene and/or a decreased or abolished activity of the AGAT enzyme which is in turn an indication of an increased mortality risk.

In the present invention, homoarginine plasma concentrations were monitored in 394 patients after acute ischemic stroke during a median follow-up of 7.4 years, and it was found that homoarginine was inversely associated with survival (HR 0.79 (0.64, 0.96); p=0.019 for each increase by 1 SD). To identify common genetic variants related to homoarginine plasma concentrations in humans, a genome-wide association (GWA) study in a population-based study (n=3,500) was conducted. Homoarginine was associated with a number of single nucleotide polymorphisms (SNPs) of AGAT (each p<10⁻⁹). This association between homoarginine and AGAT genotypes was confirmed in the stroke cohort.

In a mouse model with AGAT gene disruption (AGAT^{-/-}) guanidino acetate, creatine, and homoarginine were absent showing that AGAT is involved in the biosynthesis of this molecule. AGAT^{-/-} mice supplemented with creatine (which still lacked homoarginine) were subjected to experimental ischemic stroke by middle carotid artery occlusion (MCAO) and experienced severe strokes and impaired survival which could be preserved by homoarginine.

In addition, it was shown that transfection of the murine AGAT gene into HEK cells which do not show AGAT mRNA expression results in the formation of AGAT mRNA. Similarly, the incubation of AGAT-expressing HEK293 cells with [¹³C₆]-lysine and L-[¹⁵N₂]-arginine resulted in the formation of [¹⁵N₂¹³C₆]-homoarginine.

These data demonstrate for the first time that AGAT is involved in the formation of homoarginine in humans and mice. Both homoarginine and the AGAT genotype are related to survival after acute ischemic stroke.

### BRIEF DESCRIPTION OF THE FIGURES

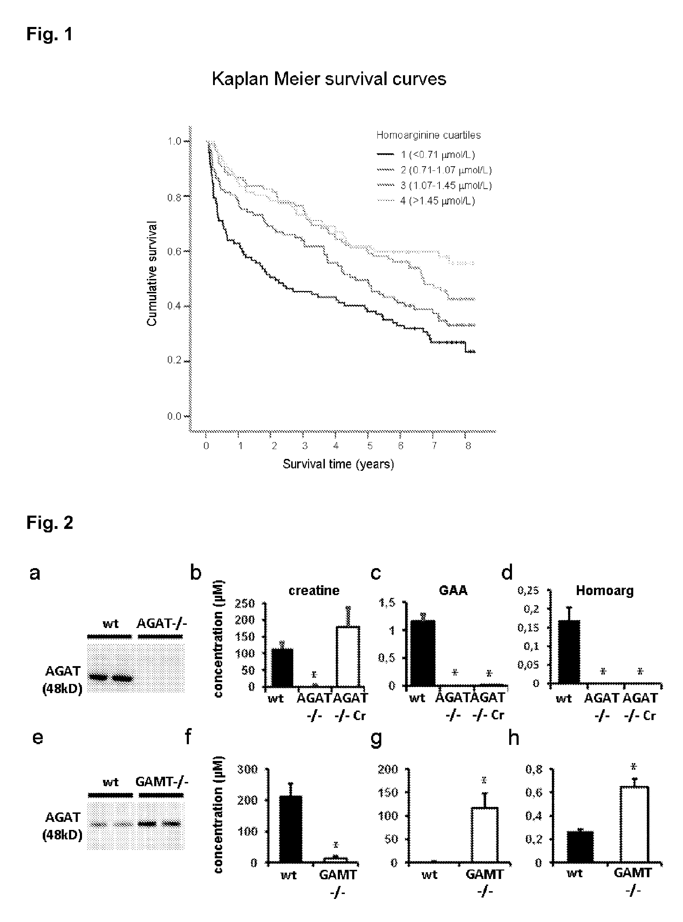

Figure 1 depicts Kaplan-Meier survival curves which indicate the relationship of quartiles of homoarginine with all-cause mortality after ischemic stroke during a median follow up of 7.4 years (p<0.001).
Figure 2 shows the characterization of AGAT^{-/-} mice and wild type littermates. Expression analysis of AGAT in kidney from (a) AGAT^{-/-} and (e) GAMT^{-/-} mice by western blot. Plasma concentrations of (b, f) creatine, (c, g) guanidino acetate (GAA), and (d, h) homoarginine (Homoarg) in (b-d) wt, AGAT^{-/-} and AGAT^{-/-} mice supplemented with 1% creatine, as well as in (f-h) wt and GAMT^{-/-} mice, *p<0.01.

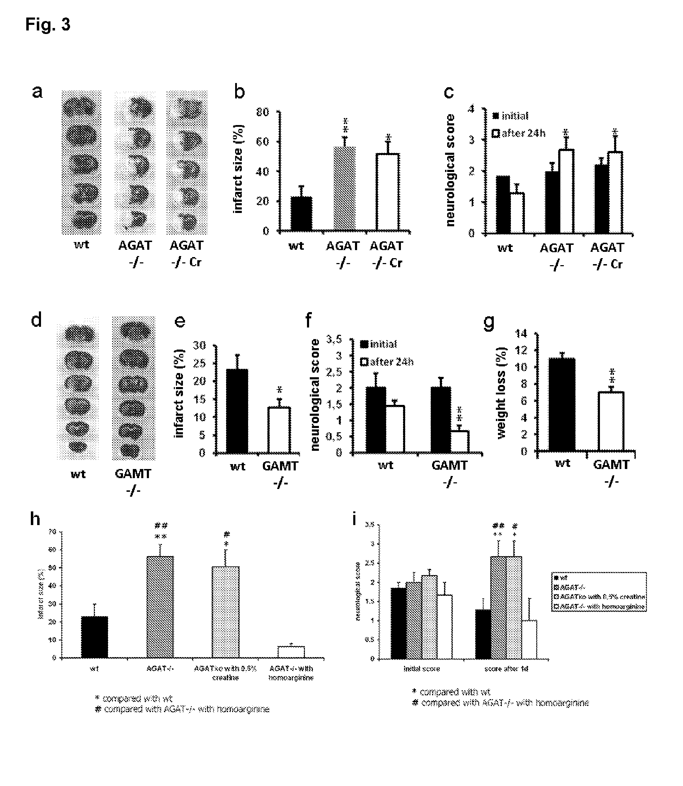

Figure 3 depicts the results obtained after temporary middle cerebral artery occlusion (MCAO) in AGAT-deficient and AGAT-overexpressing mice. AGAT^{-/-} mice with and without creatine supplementation as well as wild-type littermates were subjected to MCAO. (a, d) Representative of triphenyltetrazolium chloride (TTC)-stained brain slices. (b, c) Effects of AGAT gene deletion on cerebral ischemia in mice subjected to 30 min of MCAO. Infarct sizes (a, b) and neurological scores (c) were measured after 24 hours (n=7/6/6). Furthermore, AGAT^{-/-} mice were supplemented with homoarginine regardless of creatine substitution (n=3). GAMT^{-/-} mice and wild-type littermates were subjected to temporary occlusion of the mean carotid artery (MCAO). (d-g) Effects of GAMT gene deletion on cerebral ischemia in mice subjected to 30 min of MCAO. (d, e) Infarct size, (f) neurological score, and (g) weight loss were measured after 3 days (n=5/9). AGAT^{-/-} mice supplemented with creatine experienced severe strokes and impaired survival which could be preserved by homoarginine (h, i) (n=3 for group with homoarginine substitution).

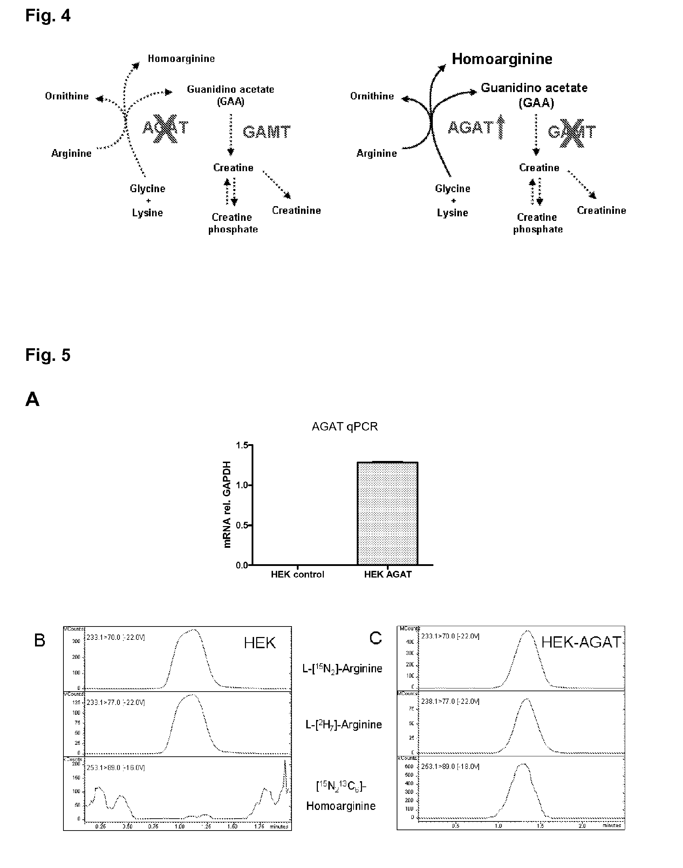

Figure 4 shows the involvement of AGAT and GAMT enzyme in the synthesis of creatine and homoarginine.
Figure 5 shows the results from transfection experiments using HEK293 cells. (A) qPCR analysis of AGAT mRNA expression in untransfected HEK293 cells (HEK control) and HEK293 cells transfected with the murine AGAT gene. (B,C) LC-MS/MS analysis of AGAT protein activity in untransfected HEK293 cells (HEK, B) and HEK293 cells transfected with the murine AGAT gene (HEK-AGAT, C). AGAT protein activity was detected by conversion of stable isotope labelled [¹³C₆]-lysine and L-[¹⁵N₂]-arginine (B,C middle traces) to [¹⁵N₂¹³C₆]-homoarginine (B,C lower traces). L-[²H₇]-arginine (B,C upper traces) was used as internal standard for measurements.

### DISCLOSURE OF THE INVENTION

The invention relates to both diagnostic and therapeutic aspects which will be described in more detail below. Specifically, the present invention provides means and methods for determining the all-cause mortality risk in a subject by assessing the expression level of the AGAT gene, the activity of the AGAT enzyme, and/or by detecting genomic alterations which are associated with a decreased expression of the AGAT gene and/or a decreased activity of the AGAT enzyme.

This means that the methods of the invention enable the prediction of an individual's general risk to die from any cause. In other words, the reason for death is not specifically limited to particular pathological conditions or diseases. Instead the all-cause mortality risk reflects the risk to die from any possible cause, wherein the fatal outcome of pathological conditions and diseases of course contribute to mortality.

According to a particular preferred embodiment of the invention, the all-cause mortality risk referred to in connection with the methods disclosed herein is the all-cause mortality risk after having experienced a cardiovascular event, i.e. the risk to die for any cause after the onset of a cardiovascular condition. A cardiovascular condition in the sense of the present invention is meant to refer to any disease affecting the cardiovascular system, including hypertension and complications thereof, ischemia and complications thereof, acute coronary syndrome (ACS), coronary artery disease (CAD), myocardial infarction, heart failure, angina, cardiac hypertrophy, arteriosclerosis, peripheral artery disease, vasospastic disease, myocarditis, pericarditis, endocarditis, stroke, such as acute ischemic stroke and/or pulmonary embolism and/or transient ischemic attack (TIA), diabetes mellitus, atrial fibrillation. The cardiovascular condition may still be present at the time of subjecting the subject to one of the methods of the present invention, or it may be cured with no detectable clinical signs of the condition or diseases remaining. In an even more preferred embodiment, the all-cause mortality referred to herein is the all-cause mortality after having experienced a stroke.

The test subject referred to in the different methods described herein will normally be a mammal, preferably a human. Generally, the subject can be of any age. Where the test subject is a human, such subject will be older than 20 years, more preferably older than 30 years, 35 years, 40 years, 45 years, 50 years, 55 years, 60 years or 65 years. For example, the human subject will be between 30 and 65 years old, e.g. between 35-60 years, 40-60 years, 45-65 years, 50-65 years, and most preferably between 55-65 years. Preferably, the test subject is currently suffering from or has previously experienced a cardiovascular event, such as a stroke.

The reference subject referred to by the methods of the invention will be of the same species as the test subject. Thus, where the test subject is a human, the reference subject will also be a human. The reference subject will preferably be a subject that is not afflicted with any health problems or conditions which are known to shorten life expectancy. Preferably, the reference subject will exhibit a homoarginine concentration in the plasma which is typical of the general population of normal, disease-free subjects. The mean plasma concentration of homoarginine in humans is in the range of 2.5±1.0 µmol/L.

In an alternative embodiment, the methods of the invention may also use a reference subject which suffers from a disease or condition which is known to affect life expectancy, e.g. a cardiovascular disease, such as a stroke. In this case, the homoarginine concentration of said reference subject in the blood or serum is lower than that measured in the general population of normal, disease-free subjects. If the AGAT expression levels and/or the AGAT enzyme activity levels essentially correspond to the levels measured in the latter reference subject, this indicates that the mortality risk in the test subject is increased.

Further, the invention provides methods of screening for novel compounds effective in increasing the expression of the AGAT gene and/or the activity of the AGAT enzyme. These compounds can be used to decrease the mortality risk of a subject, preferably after a cardiovascular event, such as a stroke.

### Diagnostic methods targeting expression levels

In a first aspect, the present invention relates to a method for predicting the all-cause mortality risk of a test subject, comprising
(a) detecting in a sample from said test subject the expression level of the L-arginine:glycine amidinotransferase (AGAT) gene;
(b) comparing the expression level of step (a) to the expression level of said gene in a reference subject;
wherein detection of an expression level of the AGAT gene which is significantly decreased in the test subject relative to the expression level in the reference subject indicates that the test subject has an increased mortality risk.

The above method is carried out with a biological sample that has been obtained from the test subject. A variety of different sample types may be used, provided that the sample contains cells which would express the AGAT gene in a normal, healthy subject, i.e. a subject having normal homoarginine concentrations in the serum. Preferably, the sample is a tissue sample, obtained from the test subject, e.g., by core needle biopsy, fine needle aspiration, and the like. Before being used in the methods of the invention, the sample can be processed to render the sample eligible for expression analysis, e.g., by cell lysis and subsequent RNA purification. In a particularly preferred embodiment, the biological sample obtained from the subject is a blood sample, and the expression level of the AGAT gene is measured, e.g., based on RNA (in particular mRNA) extracted from white blood cells, such as lymphocytes, monocytes and/or neutrophils.

For evaluating the mortality risk of the subject to be tested, the extent of expression of the gene encoding the AGAT enzyme is analyzed. The complete human AGAT gene, including intron sequences, is disclosed as SEQ ID NO:16 herein. The coding sequence of the human AGAT gene, i.e. the sequence obtained after splicing of the mRNA has occurred, is depicted in SEQ ID NO:17. Preferably, the methods of the invention which are directed to the detection of expression levels make use of the human mRNA depicted in SEQ ID NO:17. It will be appreciated that the polynucleotide sequence of the human AGAT gene can exhibit a number of sequence deviations resulting, e.g., from naturally occurring polymorphisms. Encompassed by the meaning of the term "AGAT gene" are thus also polynucleotides which result in a mRNA molecule exhibiting at least about 95%, 96%, 97%, 98%, 99% or more sequence identity to the sequence of SEQ ID NO:17. Computer programs for determining the degree of identity between nucleotide sequences are available, e.g., in the Wisconsin Sequence Analysis Package, Version 8 (available from Genetics Computer Group, Madison, USA) and include, e.g., the programs BESTFIT, FASTA and GAP, which are based on the algorithm of Smith and Waterman. These programs can be used with the standard parameters, as recommended by the manufacturer. The amino acid sequence of the human AGAT protein is depicted in SEQ ID NO:18.

Gene expression levels can be quantified by different methods which are generally known to the skilled person. The determination of the expression of the AGAT gene can be performed either at the transcriptional level or, alternatively, at the translational level. Suitable methods for monitoring expression of the AGAT gene at the transcriptional level include those which allow for the quantitative or semi-quantitative detection of mRNA levels, for example, quantitative RT-PCR (e.g., TaqMan™ RT-PCR), real-time RT-PCR, Northern-Blot analysis or other methods which are generally described, e.g., in Sambrook et al. (eds.) Molecular Cloning: A Laboratory Manual, 2nd ed., Cold Spring Harbor Laboratory Press, 1989.

The detection of expression levels at the transcriptional level usually requires as a first step the isolation of mRNA from a biological sample of the subject, e.g. from a tissue or blood sample. Methods for isolating RNA, such as mRNA, are well known in the art and are discussed in detail in the literature (see, for example, Sambrook et al. (1989), Molecular Cloning - A Laboratory Manual, Cold Spring Harbor Laboratory, Cold Spring Harbor, New York and Ausubel et al. (1994), Current Protocols in Molecular Biology, Current Protocols Publishing, New York). Such methods regularly involve the lysis of the cells or tissues obtained from the subject to be tested. Cell lysis may be performed by use of detergents which are capable of disrupting the plasma membrane of the cells. For example, buffers containing guanidine thiocyanate and/or SDS may be used for cell lysis. The methods may comprise steps in which the DNA of the cells is enzymatically digested in order to obtain pure RNA without traces of DNA which might interfere with further downstream applications, such as the monitoring of expression levels. Inhibitors of enzymes which lead to the degradation of the RNA may also be added to the lysis buffer. Kits for preparing highly purified RNA are available from several manufacturers, such as Qiagen, Ambion, Stratagene, Clontech, Invitrogen, Promega, and others.

The RNA isolated from the cell or tissue sample by use of commercial kits will normally comprise different types of RNA. Preferably, the RNA obtained from the tissue sample is total RNA comprising mRNA, transfer RNA (tRNA) and ribosomal RNA (rRNA). For the methods of the present invention, it is desirable to enrich the mRNA fraction with respect to fractions of other cellular RNAs. Preferably, the mRNA is separated from other RNA molecules. Methods for enriching or purifying mRNA are known in the art. For example, an affinity chromatography using oligo(dT) or poly(U) coupled to a solid matrix, such as a cellulose or Sephadex™ matrix can be performed, as mRNAs contain a poly(A) tail at their 3' terminus (see, for example, Ausubel et al. (1994), Current Protocols in Molecular Biology, Current Protocols Publishing, New York). Poly(A)+ mRNA which has bound to the affinity matrix can be eluted using 2 mM EDTA/0.1% SDS.

One commonly used method for detecting expression at the transcriptional level is RT-PCR. In this method, an mRNA template is transferred into cDNA by a reverse transcription (RT) reaction. The reverse transcription of the RNA template is catalyzed by a reverse transcriptase, e.g. an avilo myeloblastosis virus reverse transcriptase (AMV-RT) or a Moloney murine leukemia virus reverse transcriptase (MMLV-RT), both of which are commonly used in commercial kits of different manufacturers. Reverse transcription can be primed by specific oligonucleotide primers, or, alternatively, by oligo-dT primers. The cDNA is then used as template for a subsequent PCR reaction. In the subsequent PCR step, the cDNA is amplified by use of specific oligonucleotide primers and a polymerase enzyme, e.g., a Taq polymerase. In a preferred aspect, the RT-PCR reaction is performed as a real-time RT-PCR, which enables the detection and the simultaneous quantification of amplified DNA in real-time. Quantification occurs either as an absolute number of copies or as a relative amount normalized by use of additional gene expression products.

In a further preferred aspect, a TaqMan RT-PCR is used for determining the expression levels of the AGAT gene. The TaqMan RT-PCR is a fluorophore-based RT-PCR method which detects accumulation of a specific, amplified product during PCR. In TaqMan RT-PCR, RNA is first transferred into cDNA by a reverse transcriptase. In the subsequent PCR reaction, a single-stranded oligonucleotide probe is added which is complementary to a segment of 10-60 nucleotides within the DNA template and located between the two PCR primers. A fluorophore and a quencher dye are covalently attached to the 5' and 3' ends of the probe, respectively. Alternatively, the quencher dye can also be attached to an internal nucleotide, while the fluorophore is attached to the 5' or 3' end of the probe or vice versa. The close proximity between fluorophore and quencher dye attached to the probe inhibits fluorescence emission from the fluorophore when said fluorophore is selectively excited. During DNA synthesis in the PCR, the 5' exonuclease activity of the Taq polymerase cleaves the oligonucleotide probe which is hybridized to the template DNA, thereby spatially separating the fluorophore and the quencher dye. Fluorescence is detected during PCR cycling; it is directly proportional to the fluorophore released and the amount of DNA template present in the PCR. Every fluorophore-quencher pair known in the art can be used in the methods of the present invention. Examples of suitable fluorophores are FAM (6-carboxyfluorescin), TET (tetrachlorofluorescin) or VIC. A suitable quencher dye is TAMRA (tetramethylrhodamine). The construction and labelling of oligonucleotides to be used as probes in a TaqMan approach are described in great detail in the literature. A TaqMan approach RT-PCR reaction can be carried out using, e.g. the ABI PRISM 7700 system (Perkin-Elmer/Applied Biosystems, Foster City, Calif., USA), or the Lightcycler system (Roche Molecular Biochemicals, Manheim, Germany).

A microarray is another commonly used tool in expression profiling. A microarray refers to the orderly arrangement of spatially resolved probes, for example nucleic acid probes, on a substrate. Such arrays can contain at least one, and preferably more than one, oligonucleotide which is complementary to the AGAT gene and, thus, hybridizes to the AGAT sequence or its transcripts. The substrate is preferably a solid substrate having a surface with a plurality of probes attached in different known locations (spots). The spots on a microarray are normally either printed on the microarrays or synthesized by photo-lithography or by ink-jet printing. On a common microarray, there may be several thousands spots, wherein each of the spots may contain a high number of identical probes, such as nucleic acid fragments or oligonucleotides. Such microarrays typically have a density of at least 100 oligonucleotides or fragments per cm². In certain embodiments the arrays can have a density of about at least 500, at least 1000, at least 10,000, at least 10⁵, at least 10⁶, at least 10⁷ oligonucleotides or fragments per cm². The support can be a glass slide or membrane on the surface of which the probes are attached at fixed locations or spots.

The primers or probes used in the PCR or RT-PCR reactions mentioned herein will be designed to allow the specific hybridization to and the subsequent amplification of the target sequence within the AGAT gene. Based on the present disclosure, the skilled person will be readily able to design oligonucleotide primers and/or probes which can be used for detecting the expression of the AGAT gene. Methods for designing sequence-specific oligonucleotide primers for PCR or RT-PCR are discussed in great detail in the scientific literature, e.g. in Dieffenbach and Dveksler, "PCR Primer", A Laboratory Manual, Cold Spring Harbor Laboratory Press, New York, 2003. The parameters to be considered when designing PCR primers include, for example, the number of nucleotides, the G/C content of the primer, the melting temperature, the presence of complementary nucleotides which may lead to secondary structures, and the like. The oligonucleotides for use in the methods of the present invention preferably have a length of at least 8 nucleotides, more preferably, at least 10, 12, 14, 16, 18, 20, 22, 24, 26, 28, 30, 32, 34, 36, 38, 40, 42, 44, 46, 48 or 50 nucleotides.

The oligonucleotide primers can be prepared by any suitable technique known in the art. For example, they may be derived synthetically, for example by the phosphoamidite method. Apart from oligomers or polymers which contain the naturally occurring nucleotide bases adenine, thymine (uridine), cytosine or guanine, oligonucleotide primers of the invention may also include modified bases, such as 5-methylcytosine, 5-hydroxymethylcytosine, xanthine, hypoxanthine, 2-aminoadenine, 6-methyladenine, 6-methylguanine, 2-thiouracil, 2-thiothymine, 2-thiocytosine, 5-halouracil, 5-halocytosine, 5-propinyluracil, 5-propinyl-cytosine, 6-azouracil, 6-azocytosine, 6-azothymine, 5-uracil, 4-thiouracil, 8-thioalkyladenine, 8-hydroxyladenine, 8-thioladenine, thioalkylguanine, 8-hydroxylguanine, 8-thiolguanine, 7-methylguanine, 7-methyladenine, 8-azaguanine und 8-azaadenine, 7-deazaguanine, 7-deazaadenine, 3-deazaguanine and/or 3-deazaadenine, and the like.

Where the determination of the mRNA levels reveal that the AGAT gene expression is significantly decreased in the test subject relative to the expression level in the reference subject, this indicates that the test subject has an increased mortality risk. A significant decrease of the expression level of the AGAT gene means that the AGAT mRNA amounts found in the test subject are at least about 25%, about 30%, about 35%, about 40%, about 45%, about 50%, about 55%, about 60%, about 65%, about 70%, about 75%, about 80%, about 85%, about 90%, about 95%, or at least about 100% lower than the respective amounts in the healthy reference subject. In other words, the expression level of the AGAT gene can be decreased in the test subject by at least 2-fold, at least 5-fold, at least 10-fold, or at least 20-fold relative to the expression level in the reference subject.

The detection of expression levels at the translational level is usually performed by measuring AGAT protein levels. AGAT protein levels may be determined by any suitable method which is able to specifically detect the AGAT protein in a biological sample. The detection of the protein may be based on a molecule that binds specifically to the protein or on the separation of the protein from other proteins that are present in the sample.

Molecules that specifically bind to the AGAT protein include antibodies and antibody fragments with binding activity for the AGAT protein. Such antibodies or fragments thereof may be used for detection of the AGAT protein in a Western blot, quantitative Western blot, enzyme-linked immunosorbent-assay (ELISA), polarization analysis, (quantitative) surface plasmon resonance (SPR), or in immunohistochemical methods, including quantitative electronmicroscopic methods. Other methods which are able to detect specific binding include, for example Förster/fluorescence resonance energy transfer (FRET). Methods which allow the quantitative detection of the AGAT protein by separating the protein from other components in the biological sample include quantitative mass spectrometric methods, electrophoretic methods, such as two-dimensional gel electrophoresis, and chromatographic methods, such as size-exclusion chromatography or ion-exchange chromatography.

The amounts of the AGAT protein measured in the sample of the test subject are indicative for the mortality risk of said subject. If the protein levels reveal that the AGAT gene expression is significantly decreased in the test subject compared to the reference subject, this indicates that the test subject has an increased mortality risk. A significant decrease of the expression level of the AGAT gene means that the AGAT protein amounts found in the test subject are at least about 25%, about 30%, about 35%, about 40%, about 45%, about 50%, about 55%, about 60%, about 65%, about 70%, about 75%, about 80%, about 85%, about 90%, about 95%, or at least about 100% lower than the respective amounts in the healthy reference subject. The protein amounts in the test subject can be decreased by at least 2-fold, at least 5-fold, at least 10-fold, or at least 20-fold relative to the corresponding amounts in the reference subj ect.

### Diagnostic methods targeting enzymatic activity

In a further aspect, the invention relates to a method which compares the activity of the AGAT enzyme as measured in a sample from a test subject to a reference value that was obtained from a healthy person, i.e. a person who does not show any clinical signs for a cardiovascular disease, such as a stroke.

The method comprises
(a) detecting in a sample from said test subject the activity of the L-arginine:glycine amidinotransferase (AGAT) enzyme;
(b) comparing said activity of step (a) to the activity of said enzyme in a reference subject;
wherein detection of an activity of the AGAT enzyme which is significantly decreased in the test subject relative to the activity in the reference subject indicates that the test subject has an increased mortality risk. Preferably, the sample in which the concentration of the homoarginine is detected, is a blood sample, more preferably a blood plasma sample.

Enzymatic AGAT activity may be determined in a suitable assay, e.g. by measuring the reduction rate of educts of the AGAT reaction, preferably arginine and lysine. The educts of the reaction may be labelled for the purpose of a simplified detection of the product of the reaction. The educts may for example be labelled with stable isotopes or radioisotopes, such as ²H, ¹³C, ¹⁵N, ¹⁸O, ³H and ¹⁴C, and monitored during reaction by mass spectrometric detection or scintillation detection. This method has been previously described in detail for stable isotopes by Maas et al. (2007), Chromatogr B Analyt Technol Biomed Life Sci., 851:220-8). Such an enzyme activity assay is based on scintillation detection for radioisotopes and has been previously described in detail by MacAllister et al. (1996), Br J Pharmacol., 119:1533-40). In a preferred embodiment, the assay for determining the activity of the AGAT enzyme measures the formation of [¹⁵N₂¹³C₆]-homoarginine from the educts [¹³C₆]-lysine and L-[¹⁵N₂]-arginine in the presence of AGAT.The enzyme activity assays for detecting enzymatic AGAT activity are preferably carried out with the human AGAT enzyme depicted in SEQ ID NO:18. Alternatively, an enzymatically active fragment or homolog of the human enzyme shown in SEQ ID NO:18 may be used. As used herein, a homolog of the sequence of SEQ ID NO:18 refers to a polypeptide molecule having a high degree of sequence identity with the amino acid sequence of SEQ ID NO:18. The amino acid identity will be at least about 75%, 80%, 85%, 90%, 91%, 92%, 93%, 94%, 95%, 96%, 97%, 98% or 99% when compared in an optimal alignment, for example, by the program BESTFIT using standard parameters.

The assays used according to the present invention may also be based on one or more enzymatically active fragments of the human AGAT enzyme shown in SEQ ID NO:18 or on enzymatically active fragments of the above-mentioned homologs. Enzymatically active fragments of the sequence shown in SEQ ID NO:18 or its homologs are polypeptides that differ from the amino acid sequence shown in SEQ ID NO:18 (or from the respective homolog sequence) by the absence of one or more amino acids at the N-terminus and/or the C-terminus of the polypeptide. For example, a fragment of the sequence of SEQ ID NO:18 may differ from the sequence of SEQ ID NO:18 by the lack of about 5, 10, 15, 20, 25, 30, 35, 40, 45 or 50 amino acids at the N-terminus and/or the C-terminus, provided that such fragment retains at least a part of the enzymatic activity of the original full-length enzyme depicted in SEQ ID NO:18. Likewise, a fragment of a homolog of SEQ ID NO:18 may differ from said homolog sequence by the lack of about 5, 10, 15, 20, 25, 30, 35, 40, 45 or 50 amino acids at the N-terminus and/or the C-terminus, provided that the fragment is still enzymatically active.

A significant decrease in the AGAT enzyme activity means that the AGAT activity levels found in the test subject are at least about 25%, about 30%, about 35%, about 40%, about 45%, about 50%, about 55%, about 60%, about 65%, about 70%, about 75%, about 80%, about 85%, about 90%, about 95%, or at least about 100% lower than the respective levels in the reference subject. The activity levels in the test subject can be decreased by at least 2-fold, at least 5-fold, at least 10-fold, or at least 20-fold relative to the corresponding levels in the reference subject.

### Diagnostic methods targeting homoarginine plasma levels

In still a further aspect, the invention relates to a method for predicting the all-cause mortality risk of a test subject, comprising
(a) detecting in a sample from said test subject the concentration of homoarginine;
(b) comparing the concentration of step (a) to the concentration of homoarginine in a sample of a reference subject;
wherein detection of a homoarginine concentration which is significantly decreased in the test subject relative to the concentration in the reference subject indicates that the test subject has an increased mortality risk. The sample in which the concentration of the homoarginine is detected preferably is a blood sample, more preferably a blood plasma sample.

The concentration of homoarginine in the sample, e.g. in a blood plasma sample, can be detected by several methods known in the art. These methods include, for example, immunoassay-based methods, such as enzyme-linked immunosorbent-assay (ELISA) and radioimmunoassay (RIA). Chromatographic methods for the detection of homoarginine in the blood plasma include for example high performance-liquid chromatography (HPLC), tandem liquid chromatography-mass spectrometry (LC-MS/MS), tandem gas chromatography-mass spectrometry (GC-MS/MS), and the like. In a particularly preferred embodiment, the concentration of homoarginine is detected by LS-MS/MS in human plasma. In a particularly preferred embodiment, the concentration of homoarginine is detected by high performance liquid chromatography and fluorescence detection after derivatization with o-phthalaldehyde as described previously (Teerlink T, et al. (2002), Anal Biochem. 303:131-7).

The reference subject will preferably exhibit a homoarginine concentration in the plasma which is typical of the general population of normal, disease-free subjects. In humans, the mean plasma concentration of homoarginine is in the range of 2.5±1.0 µmol/L. The homoarginine concentration is significantly decreased in the test subject, if the homoarginine concentration in the test subject is lower than 1.5 µmol/L, preferably lower than 1.0 µmol/L, 0.75 µmol/L, or 0.5 µmol/L.

### Diagnostic methods targeting genomic DNA

In another aspect, the present invention relates to methods for assessing the all-cause mortality risk of the subject by detecting alterations in the genome of a subject that result in a decreased expression of the AGAT gene and/or a reduced activity of the AGAT enzyme.

Specifically, the present invention provides a method for predicting the all-cause mortality risk of a test subject, comprising detecting in a biological sample a genetic alteration in the genome of said test subject, wherein said genetic alteration is associated with a decreased expression of the AGAT gene and/or with a decreased activity of the AGAT enzyme. The detection of such a genetic alteration indicates that the test subject has an increased mortality risk.

The genetic alteration referred to herein may be any kind of alteration in the genome of the test subject that is capable of interfering with the expression of the AGAT gene, such as inactivating insertion, deletions of one or more nucleotides in the coding region, mutations of the promoter structure, single nucleotide polymorphisms, loss of gene fragments and the like. The genetic alteration can be located within intronic sequences or the coding sequence of the AGAT gene or in a region flanking said coding sequence. The alteration may either completely or partially decrease the expression of the AGAT gene. Alternatively, the alteration may completely or partially inactivate the AGAT enzyme, i.e. lead to a decreased activity of the enzyme compared to the wild type enzyme.

In a preferred embodiment, the detection of the genetic alteration which is associated with a decreased expression of the AGAT gene and/or with a decreased activity of the AGAT enzyme comprises the detection of a single nucleotide polymorphism (SNP). As used herein, a SNP refers to a nucleotide position in a nucleic acid, such as a DNA or RNA, which is occupied by alternative nucleotides in different alleles in a population. The position of the SNP is usually flanked by highly conserved sequences of the allele. An individual, such as a human, normally has two alleles and therefore may be homozygous or heterozygous with respect to a given allele at a particular SNP position. In a biallelic organism, for example in a human, a single nucleotide polymorphism can result in three different genotypes. For example, an A/C SNP marker can result in a homozygous genotype "AA", a homozygous genotype "CC" or a heterozygous genotype "AC". Most SNPs have only two alleles, i.e. one group of the population exhibits a specific nucleotide at the SNP position, whereas the other group exhibits a different nucleotide at the same position.

A SNP can be located in a coding region of the AGAT gene, in a non-coding region or in an intergenic region. Where the SNP is located in the coding region of the AGAT gene, it may be synonymous or non-synonymous. A synonymous or silent nucleotide substitution is one that due to the degeneracy of the genetic code does not result in an amino acid change. In contrast, a non-synonymous nucleotide substitution yields in an amino acid substitution and frequently gives rise to an altered expression product. Alternatively, it may lead to a premature termination of the translation process if a stop codon is formed by the substitution at the polymorphic site. It is also possible that the substitution destroys a naturally occurring stop codon at the polymorphic site which leads to an extended read-through expression product. In contrast, where a SNP is not in a coding region of the genome, it may nevertheless affect protein expression, for example by influencing RNA splicing or DNA binding to transcription factors, and the like.

The present invention provides a number of SNPs in the vicinity of the AGAT gene which have been found to be associated with a low homoarginine concentration in the serum of individuals, and therefore with an increased mortality risk. The SNPs are listed in the below table. The nucleotides in brackets indicate the polymorphism. The first nucleotide represents the major allele which is found in the risk allele, i.e. the nucleotide which has been identified in persons having a significantly lower homoarginine plasma concentration. The second nucleotide (minor allele) in brackets is the nucleotide which is considered the non-risk allele.

**Table 1: SNPs associated with decreased homoarginine plasma concentration**

| | |
|---|---|
| rs2467864 | ACCTGCCTCTCCCTGAGTCTTCCTCT[A/G]TTTAGTTAAGGTAATTCCATCCCTC (SEQ ID NO:1) |
| rs10519022 | AGATAATTTCAGATAGCAATAAGTAC[T/C]AGAAAGGTGTAAAACTTGGTAATGG (SEQ ID NO:2) |
| rs11852800 | GCAGCCCTTTTGCAACTTTTAAACAG[C/T]GATGAGAATTCTGGCCCTTTGTCTT (SEQ ID NO:3) |
| rs1153858 | CAAATGTGTATAAATCTATAGGGGAT[C/T]GTAAACTGAAGGAACTAATGCCTGA (SEQ ID NO:4) |
| rs9783731 | TTTTTCTCCATTTCACTGAGGTTCTG[T/C]AGTAGGCAATGCTGTCTTAGCATGT (SEQ ID NO:5) |
| rs12439274 | CCTTCTCAGTGCACCTGATTATGTCC[T/C]TGAGTCTCAGAGATTTTTTGCTTCC (SEQ ID NO:6) |
| rs1554521 | CACCAAGCTAGGTTCCAGCGTAACTG[A/T]TGTGAACCTGGATATTGAACTAACC (SEQ ID NO:7) |
| rs1145091 | GAAAATATTTTGCTAAGCGATAGAAG[T/C]TCATCACAAAAGACCACTTACTGAA (SEQ ID NO:8) |
| rs1719247 | TCCACATACACCTGATGAAAAGCTTA[C/T]GACACAGGAAGACATCAGCTCAACA (SEQ ID NO:9) |
| rs4395020 | TATATACCCTAATGTGCACCAATCCA[A/C]ACAACTGGATAGATTTCTCAAGCAG (SEQ ID NO:10) |
| rs4775911 | CCCAAGAGAAGTGAAAACATATGCCT[A/C]CGCAGAAACTTGTATGTAAGTGTTC (SEQ ID NO:11) |
| rs1145077 | CATTTCTTGAATAGATTTGTTTGTGT[G/T]TATAAGCTAGCCAGTGGTTTAGTTA (SEQ ID NO:12) |
| rs1346267 | GTAGATTAAATAATGTTAATCTTTCC[A/G]GCATTTCCATTTTTGTGTTATGTTC (SEQ ID NO:13) |
| rs1153860 | GGTATCAAAACCTCTCATTTCTTTCA[C/T]TGAGGCTTTAACAGTGCTAGGCTGG (SEQ ID NO:14) |
| rs1288775 | TGATCTTTGGGAAAATAATGCCCTCC[A/T]TGCTTCTGGTAAAATGGCCAGTACT (SEQ ID NO:15) |

In a preferred embodiment, the diagnostic method of the invention which makes use of polymorphisms in the genomic DNA of individuals suffering from an increased mortality risk comprises the step of detecting the presence or absence of at least one nucleotide exchange from a non-risk to a risk allele depicted in Table 1 in a nucleic acid sample of the test subject, wherein a change from the non-risk allelic form to the risk allelic form is indicative for an increased mortality risk. The detection of a homozygous genotype of the risk allele in a subject generally indicates a higher risk than the detection of the corresponding heterozygous genotype.

The detection of a specific nucleotide at a SNP site in a polynucleotide is commonly referred to as genotyping of the SNP. Numerous methods are described in the prior art which provide for genotyping of SNPs in genomic sequences. See for example, Chen et al, Pharmacogenomics J. (2003), 3(2):77-96; Kwok et al., Curr. Issues Mol. Biol. (2003), 5(2):43-60; Kwok, Annu. Rev. Genomics Hum. Genet. (2001), 2:235-58; Shi, Am. J. Pharmacogenomics (2002), 2(3):197-205. For example, methods for SNP genotyping that can be used according to the present invention include TaqMan assays, molecular beacon assays, nucleic acid microarrays, primer extension techniques and oligonucleotide ligation assays. These methods may be used in combination with different labeling techniques, including fluorescence, luminescence or chemiluminescence labeling, and the like. Preferably, the genotyping methods are suitable for a high throughput format and accessible to automation.

In a simple embodiment, genomic DNA is amplified by PCR methods. For this, genomic DNA is firstly isolated from a sample of the test subject. A variety of different sample types may be used, provided that the sample contains cells containing genomic DNA of the test subject. The sample may be a tissue sample obtained from the test subject, e.g., by core needle biopsy, fine needle aspiration, and the like. In a preferred embodiment, the biological sample is a blood sample, and the genomic DNA is extracted from white blood cells, such as lymphocytes, monocytes and/or neutrophils. Several methods for isolating genomic DNA from eukaryotic cells or tissues have been described in the art. The genomic DNA may for example be isolated as described by Sambrook, et al., Molecular Cloning: A Laboratory Manual, CSH Press, 2nd edition (1989). More conveniently, commercially available kits can be used for isolating genomic DNA, for example, the QIAamp DNA mini kit (Qiagen) or the ChargeSwitch® Genomic DNA Purification Kit (Invitrogen). Methods for isolating DNA usually involve the lysis of the cells of the cellular or tissue sample obtained from the test subject. Cell lysis may be performed by disrupting the plasma membrane of the cells, for example, by boiling the sample or by use of detergents such as SDS or similar compounds. The RNA of the sample is normally digested by using RNAse. Protein contaminants can be readily degraded by incubating the sample with proteinase K. Genomic DNA can be recovered by alcohol precipitation or by binding to an anion exchange column.

PCR-based methods are then used to amplify a nucleic acid sequence comprising the putative SNP site of interest. Briefly, PCR primers which specifically hybridize to upstream and downstream regions of the SNP site in the nucleic acid to be investigated can be used for the amplification. The PCR products obtained in this manner can then be subjected to common sequencing techniques, e.g. by a method based on the chain termination technique described by Sanger (J. Molecular. Biol. 94:441 (1975)). The PCR used for SNP genotyping may be a multiplex PCR. Such a multiplex PCR is performed by using a plurality of primers and genomic DNA as template for amplifying the nucleic acid sequences containing the SNP(s) to be detected.

In a preferred embodiment of the present invention, a TaqMan^{™} PCR approach is used for SNP genotyping. The basic principles of the TaqMan^{™} PCR approach are discussed elsewhere herein. It is preferred to use differently labeled probes in the same TaqMan^{™} assay. For example, one TaqMan^{™} probe specific for the risk allele of the SNP is labeled with FAM and another TaqMan^{™} probe that is specific for the non-risk allele is labeled with VIC. In such a set-up, fluorescence of FAM and no fluorescence of VIC would indicate the presence of the risk allele, whereas fluorescence of VIC and no fluorescence of FAM would be indicative for the presence of the non-risk allele. Fluorescence of both, FAM and VIC, would be indicative for a heterozygous state. The present invention also contemplates modifications of the TaqMan^{™} method, for example, assays that use molecular beacons.

A further method for detecting a SNP in a polynucleotide sequence is described, for example, in US 4,988,617. In this method, the occurrence of a specific nucleotide is determined by an oligonucleotide ligation assay using a pair of oligonucleotide probes that selectively hybridize immediately upstream and downstream of the polymorphic site. Upon hybridization, one of the terminal nucleotides of the oligonucleotides is aligned to the site of the putative polymorphism. Where the terminal nucleotide of the probe is complementary to the nucleotide at the polymorphic site, hybridization of the oligonucleotide to the polynucleotide that contains the polymorphic site will include the terminal nucleotide of said oligonucleotide. In this case, the addition of a ligase, such as a T4 ligase, will link both oligonucleotide probes to each other. In contrast, where the terminal nucleotide of the probe is not complementary to the nucleotide at the polymorphic site, no ligation of the oligonucleotide to the polynucleotide containing the polymorphic site will occur. The ligation product derived from the linkage of the two oligonucleotides can be detected in a subsequent step, for example by a PCR reaction. Several test systems are currently available which are based on oligonucleotide ligation.

Single base extension methods can also be used for the detection of SNPs. Single base extension methods are described, e.g., in US 5,846,710, US 6,004,744, US 5,888,819 and US 5,856,092. Briefly, these methods are based on the hybridization of a primer that is complementary to a target sequence such that the 3' end of the primer is adjacent to but does not span the site of the putative SNP in the target sequence. The primer comprises a sequence which is complementary to the target polynucleotide terminating at the base that is immediately adjacent 5' to the polymorphic site. The hybridization is performed in the presence of one or more labeled nucleotides complementary to base(s) that may occupy the site of potential variation. For example, for a biallelic polymorphism two differentially labeled nucleotides can be used. For a tetra-allelic polymorphism four differentially labeled nucleotides can be used. Preferably, the labeled nucleotides are dideoxynucleotides. Hybridization is performed under conditions permitting primer extension if a nucleotide complementary to a base occupying the site of variation in the target sequence is present. Extension incorporates a labeled nucleotide thereby generating a labeled extended primer. If multiple differentially labeled nucleotides are used with a heterozygous target, multiple differentially labeled extended primers are obtained. Extended primers are detected providing an indication of which base(s) occupy the polymorphic site in the target sequence.

Another embodiment of the invention refers to genotyping by an invader assay (Olivier M., Mutat Res. (2005), 573(1-2), 103-10). In the invader assay, a cleavase (e.g., the flap endonuclease FEN) is combined with two specific oligonucleotide probes that together with the target DNA can form a tripartite structure recognized by the cleavase. The first probe, called the invader oligonucleotide is complementary to the 3' end of the target DNA. The last base of the invader oligonucleotide is a non-matching base that overlaps the SNP nucleotide in the target DNA. The second probe is an allele-specific probe which is complementary to the 5' end of the target DNA, but also extends past the 3' side of the SNP nucleotide. The allele-specific probe will contain a base complementary to the SNP nucleotide. If the target DNA contains the desired allele, the invader and allele-specific probes will bind to the target DNA forming the tripartite structure. This structure is recognized by cleavase, which will cleave and release the 3' end of the allele-specific probe. If the SNP nucleotide in the target DNA is not complementary to the allele-specific probe (risk or non-risk allele), the correct tripartite structure is not formed and no cleavage occurs. The invader assay can be coupled with a fluorescence resonance energy transfer (FRET) system to detect the cleavage. In this case, a quencher molecule is attached to the 3' end and a fluorophore is attached to the 5' end of the allele-specific probe. If cleavage occurs, the fluorophore will be separated from the quencher molecule generating a detectable signal.

According to another embodiment, the genotyping of the SNPs is carried out by a hybridization assay using a suitable probe or suitable probes. Any standard hybridization assay known to the person skilled in the art can be used in the methods of the present invention. In a preferred embodiment a dynamic allele-specific hybridization approach is used. This approach is based on the differences in the melting temperature in DNA that results from the instability of mismatched base pairs. In the first step of dynamic allele-specific hybridization, a genomic fragment comprising the SNP is amplified and attached to a bead through a PCR reaction with a biotinylated primer. In the second step, the amplified product is attached to a streptavidin column and washed (e.g. with NaOH) to remove the nucleic acids which are not biotinylated. An allele specific oligonucleotide is then added in the presence of a molecule that emits fluorescence when bound to double-stranded DNA. The intensity is then measured as temperature is increased until the Tm (melting temperature) can be determined. If the probe is specific for the non-risk allele of the SNP, occurrence of the risk allele will result in a lower than expected Tm and therefore, the risk allele can be detected (Howell W., et al., Nat. Biotechnol. (2005), 17(1), 87-8).

### Screening for compounds for decreasing the mortality risk

The insight that a decreased expression of the AGAT gene and/or a decreased activity of the AGAT enzyme impair the mortality risk of a subject allows the design of screening assays which identify pharmaceutically active compounds which are effective in decreasing the mortality risk of a subject. The present invention thus also provides novel methods for screening for compositions which modulate the mortality risk of a subject. The screening methods comprise the step of monitoring the expression level of the AGAT gene or, alternatively, the activity of the AGAT enzyme in the presence of candidate compounds. Any increase in the expression level of the AGAT gene and/or the activity of the AGAT enzyme in the presence of a candidate compound relative to the expression level and/or the enzymatic activity in the absence of said candidate compound is an indication that the compound is effective in decreasing the mortality risk by increasing the homoarginine concentration in the plasma of an individual.

Thus, the invention provides an *in vitro* method for identifying a pharmaceutically active compound which decreases the mortality risk of a subject, preferably a human subject, comprising
(a) contacting a candidate compound with a cell, preferably a human cell, that expresses the L-arginine:glycine amidinotransferase (AGAT) gene;
(b) detecting whether said candidate compound alters the expression level of the AGAT gene;
wherein an increase of the expression level of the AGAT gene in the presence of the compound (relative to the expression level in the absence of said compound) indicates a pharmaceutically active compound.

In another aspect, the invention provides an *in vitro* method for identifying a pharmaceutically active compound which decreases the mortality risk of a subject, preferably a human subject, comprising
(a) contacting a candidate compound with a L-arginine:glycine amidinotransferase (AGAT) enzyme;
(b) detecting whether said candidate compound alters the activity of the AGAT enzyme;
wherein an increase of activity of the AGAT enzyme in the presence of the compound (relative to the activity in the absence of said compound) indicates a pharmaceutically active compound.

A number of different assay designs to evaluate the effects of candidate compounds on gene expression or enzyme activity may be used. The screening methods are preferably carried out as high throughput screening techniques which allow for the examination of thousands of dif ferent compounds in a short period of time. High throughput screening techniques are described in detail in the prior art. The compounds identified by the screening methods can be validated in animal models, such as mouse models to confirm their activity *in vivo*.

The candidate compounds used in the screening methods of the present invention can include all different types of organic or inorganic molecules, including peptides, oligo- or polysaccharides, fatty acids, steroids, and the like. Typically, the candidate compounds will be small molecules with less than about 2,500 daltons, less than 2000 daltons, less than 1500 daltons, less than 1000 daltons, or less than 500 daltons. Candidate compounds for use in screening methods can be provided in the form of libraries which comprise a high number of synthetic or natural compounds.

In a preferred embodiment, a candidate compound is considered potentially active if it increases the AGAT expression level or enzyme activity by at least 10%, 20%, 30%, 40%, 50%, 60%, 70%, 80%, 90%, 100%, 150%, 200%, 250%, or greater compared to the expression or activity in the absence of the compound. In some embodiments the increase in the AGAT expression level or enzyme activity will be 300%, 400%, 500%, 600%, 700%, 800%, 900% or even 1000% or greater compared to the expression or activity in the absence of the compound.

### Therapeutic approaches for decreasing the mortality risk

Apart from the predictive methods described above, the present invention also provides a method for decreasing the mortality risk of a subject, comprising administering to said subject a composition or compound which is effective in increasing the expression of the AGAT gene and/or the activity of the AGAT enzyme in said subject. The invention therefore also relates to a composition or compound which is effective in increasing the expression of the AGAT gene and/or the activity of the AGAT enzyme for use in a method of decreasing the mortality risk of a subject.

One way of complementing a pathologically decreased expression of the AGAT gene and/or a pathologically decreased activity of the AGAT activity in a subject is to provide said subject with a functional copy of the AGAT gene, e.g. by gene therapy. A gene therapy approach facilitates the insertion of a functional gene into an unspecific or specific location of the host genome. For this purpose, the AGAT gene is cloned into a viral or non-viral vector. The AGAT gene may be operably linked to a promoter element and, optionally, to an enhancer element. A suitable promoter is, e.g., the immediate early promoter of the cytomegalovirus (CMV). The promoters and enhancers may further be selected from those known in the art, such as the promoters and enhancers of the LTR of Rous sarcoma virus, the TK gene of HSV-1, the early or late promoters of SV40, the adenovirus major late promoter (MLP), phosphoglycerate kinase genes, metallothionein genes, a-1 antitrypsin genes, albumin genes, collagenase genes, elastase I genes, β-actin genes, β-globin genes, γ-globin genes, α-fetoprotein genes, and muscle creatin kinase genes.

Where the subject to be treated by gene therapy is a human, the vector to be administered typically is a viral vector. Typically, viral vectors are able to infect the target cells and introduce the gene of interest into the cell. The viral vectors may either be endogenously able to infect the target cell or be engineered to do so. Such engineering may be that the viral vector contains a ligand molecule in its viral capsid and/or envelope which binds to surface molecules of the target cells.

Suitable viral vectors for use in the present invention are recombinant DNA or RNA viruses, more preferably replication-deficient viruses, and include, e.g., detoxified retrovirus, adenovirus, adeno-associated virus (AAV), herpes virus, poxvirus, vaccinia virus, poliovirus, Sindbis virus, polyomavirus, such as simian virus 40 (SV40), human immunodeficiency virus (HIV), and others.

Adenoviruses are particularly preferred, as the transduction efficiency is typically higher with adenoviruses compared to other viruses. The adenovirus may be a human adenovirus type 5 (hAd5) vector, an E1-deleted and/or an E3-deleted adenovirus. For example, an adenoviral vector can be constructed by the rescue recombination technique as described in McGrory, et al. (1988), Virology 163:614-617. Briefly, the transgene of interest is cloned into a shuttle vector that contains a promoter, a polylinker and flanking adenovirus sequences from which E1A/E1B genes have been deleted. Suitable shuttle vectors include, e.g., the plasmid "pAC1" " (McGrory, et al. (1988), Virology 163:614-617) which encodes portions of the left end of the human adenovirus 5 genome but which lacks the early protein region comprising E1A and E1B sequences that are essential for viral replication. Another suitable shuttle plasmid is "ACCMVPLPA" (Gomez-Foix et al. (1992), J. Biol. Chem. 267: 25129-25134) which contains a polylinker, CMV promoter and SV40 polyadenylation signal flanked by partial adenovirus sequences from which the E1A/E1B genes have been deleted. The shuttle plasmid can be co-transfected, e.g., by lipofection or calcium-phosphate-transfection, along with a plasmid comprising the entire human adenovirus 5 genome with a length that is too large to be encapsidated into suitable host cells (e.g., human 293 cells). In a subset of cells "rescue recombination" between the shuttle vector and the helper plasmid will occur, creating a plasmid which contains the gene of interest in the place of the E1A/E1B genes and of the additional sequences which previously rendered the plasmid too large to be encapsidated. This can be monitored, e.g., with the beta-galactosidase/x-gal system which is well known in the art. The resulting plasmid of interest will be small enough to be encapsidated but replication deficient (see, e.g., Giordano et al. (1996), Nature Medicine 2: 534-539).

In another embodiment, the viral vector may be a poxvirus, e.g. a vaccinia virus, preferably an attenuated vaccinia virus, an avipox virus or an attenuated avipox virus, or a swinepox, raccoonpox, camelpox, or myxomatosis virus. In a vaccinia virus, the insertion site or sites for the gene of interest may be advantageously at the thymidine kinase (TK) gene or insertion site, the hemagglutinin (HA) gene or insertion site, or the region encoding the inclusion body of the A type (ATI). In yet another embodiment the viral vector may be a herpes virus such as a canine herpes virus (CHV) or a feline herpes virus (FHV). Suitable insertion sites for the gene of interest are known in the art.

Recombinant viral vectors can be plaque-purified according to standard techniques. For example, recombinant adenoviral vectors can be propagated in human 293 cells (which provide E1A and E1B functions *in trans*) to titers in the range of 10⁷-10¹³ viral particles/mL). Prior to *in vivo* application viral vectors may be desalted by gel filtration methods, such as Sepharose columns, and purification by subsequent filtering. Purification reduces potential deleterious effects in the subject to which the vectors are administered. The administered virus is substantially free of wild-type and replication-competent virus. The purity of the virus can be proven by suitable methods, such as PCR.

Non-viral expression vectors may also be used for introducing a functional AGAT gene into a human subject. Suitable expression vectors permit the *in vivo* expression of the AGAT gene in the target cell. Examples for non-viral expression vectors include vectors such as pCAGGS (Niwa et al. (1991), Gene, 108: 193-200), pBK-CMV, pcDNA3.1, pZeoSV (Invitrogen, Stratagene). These vectors may be administered, for example, by direct injection or non-invasive catheter or injector methods. Alternatively, target cells that have been removed from a subject, for example, by a biopsy procedure, may be transfected with the vector construct in an *ex vivo* procedure. The cells can then be implanted or otherwise administered into a subject, preferably into the subject from whom they were obtained. Suitable methods for the transfer of non-viral vectors into target cells are for example the lipofection method, calcium-phosphate co-precipitation method, DEAE-dextran method and direct DNA introduction methods using micro-glass tubes and the like.

Prior to the introduction of the vector, the target cells and/or tissues may be treated with a permeabilization agent selected from the group consisting of SDS and SLS and their derivatives, phosphatidylcholine, streptolysins, equinatoxins, methyl-beta-cyclodextrin, sodium caprate, decanoylcamitine, tartaric acid, lysolecithin, polyoxyethylenesorbitan monolaurate, octylphenoxy polyethoxy ethanol, t-octylphenoxypolyethoxyethanol (Triton X-100), alamethicin, Saponin, non-ionic detergents (NPs, such as NP-40) or Tween and its derivatives. Target cells may also be permeabilized by ultrasonic shock, osmotic shock or electric pulses.

Another therapeutic approach for decreasing the mortality risk of a subject is to administer to a subject in need thereof a compound, such as a small molecule, which is effective in increasing the expression of the AGAT gene and/or the activity of the AGAT enzyme in order to decrease the mortality risk. Preferably, the compound has been obtained by screening of a library, such as a small molecule library, as described elsewhere herein. The compounds which are identified by applying the screening methods disclosed herein may be further modified by molecular modelling approaches and subsequent chemical synthesis of the desired molecule. In this way, the binding affinity for AGAT, the potency to increase the AGAT expression and/or activity or other physiological properties of the compound may be improved. Suitable methods to assist in a strategic modification of a compound include the determination of the three-dimensional structure of the compound in complex with its binding partner by Nuclear-Magnetic Resonance (NMR) or X-ray crystallography and subsequent computer-based modelling of suitable compound-modifications. Strategically modified compounds will be synthesized and also tested in above described methods.

In addition, several compounds are known to enhance AGAT expression and are therefore potentially useful in the therapeutic methods referred to herein. Compounds which have been shown to increase AGAT expression are, for example, growth hormone and thyroxine. (McGuire et al. (1980), J. Biol. Chem., 255 (3):1152-1159). Additional useful compounds which increase AGAT expression are testosterone or methyltestosterone (Wyss et al. (2000), Physiol Reviews, 80: 1107-1213). Thus, the invention also relates to therapeutic approaches for decreasing the mortality risk of a subject, wherein a compound selected from the group consisting of testosterone, methyltestosterone, growth hormone and thyroxine is administered in an amount which is effective for increasing expression of the AGAT gene in a subject. Another possibility of enhancing AGAT expression is to decrease the serum concentration of creatine. A reduction in the creatine serum concentration will increase AGAT expression (Wyss et al. (2000), Physiol Reviews, 80: 1107-1213).

Finally, the invention also relates to the therapeutic use of homoarginine or a homoarginine derivative for decreasing the mortality risk of a subject. The present invention demonstrates in a knock-out mouse model that decreased plasma concentrations of homoarginine are associated with larger stroke area volumes (see Examples below). On the other hand, mice with increased homoarginine levels relative to wild-type mice were protected. Accordingly, the supplementation of a subject suffering from a pathologically low homoarginine concentration in the plasma with homoarginine is effective in protecting said subject against cardiovascular events, such as stroke, thereby decreasing the mortality risk of a subject.

The present invention provides homoarginine-containing pharmaceutical compositions which are useful for decreasing the mortality risk of a subject. The preparation of pharmaceutical compositions containing amino acids or amino acid derivatives is well known by those working in the field of pharmaceutics. Typically, such compositions are prepared either as liquid solutions, powders or suspensions. The homoarginine-containing composition of the invention can include commonly used pharmaceutically acceptable excipients, such as diluents and carriers. In particular, the composition comprises a pharmaceutically acceptable carrier, e.g., water, saline, Ringer's Solutions, or dextrose solution. Further examples of suitable carriers are described in standard textbooks, for example, in "Remington's Pharmaceutical Sciences", Mack Pub. Co., New Jersey (1991). In addition to the carrier, the composition may also contain wetting agents, emulsifying agents, pH buffering agents, stabilizers, dyes and the like.

The pharmaceutical composition provided by the present invention will be formulated to be compatible with the intended route of administration. Different routes of administration are feasible for providing the homoarginine-containing composition to a subject in need thereof. The pharmaceutical composition may be formulated for parenteral administration, for example, for intravenous, intrathecal, intradermal, subcutaneous, or rectal administration. The composition can be enclosed, e.g. in ampoules, disposable syringes or multiple dose vials made of glass or plastic.

The pharmaceutical composition of the invention may be formulated for oral administration, for example, in the form of granules, powders, tablets, pills, or capsules. The preparation of solid dosage forms can be achieved by mixing homoarginine with an appropriate edible carrier, such as microcrystalline cellulose, methylcellulose, hydroxypropyl methylcellulose, casein, albumin, mannit, dextran, saccharose, lactose, sorbitol, agar, alginate, pectine, collagen, starch or gelatine. The solid dosage form may also include surfactants, binders, thickeners, diluents, lubricants, disintegrants, and glidants. Further, the solid dosage form can comprise antioxidants (for example, ascorbic acid, tocopherol or cysteine), preservatives (for example, parabene), coloring or flavoring agents, and the like. Mixing of the above components may involve dry granulation, wet granulation, or direct compression.

The pharmaceutical composition of the invention will comprise a therapeutically effective amount of homoarginine. A therapeutically effective amount of homoarginine is typically an amount such that, when administered in a physiologically tolerable composition, is sufficient to achieve a plasma concentration in the patient from about 0.2 µg/ mL to about 200 µg/ mL, preferably from about 1 µg/ mL to about 100 µg/ mL, wherein 50 µg/ mL is particularly preferred. Therefore, according to a preferred embodiment, the dosage per body weight can vary from about 0.15 mg per kg body weight of the subject to about 400 mg per kg body weight of the subject, and preferably from about 0.5 mg per kg body weight of the subject to about 200 mg per kg body weight of the subject, used in one or more dose administrations daily. Thus, according to a preferred embodiment of the invention, the therapeutically effective amount of homoarginine ranges from 0.15 mg per kg body weight of the subject to about 400 mg per kg body weight of the subject. Homoarginine can be orally supplemented in a manner similar to that previously described for arginine, or lysine (as the educt of the reaction catalyzed by AGAT) can be supplemented in a likewise manner previously described for citrulline (the educt of the reactions catalyzed by urea cycle to form arginine). This has been performed exemplary in a clinical investigation in humans for arginine and citrulline (Schwedhelm E, et al. (2008) Br J Clin Pharmacol., 65:51-9.).

### EXAMPLES

The aim of the experiments described herein was to evaluate the association of homoarginine levels with the outcome in patients after acute ischemic stroke and, at the same time, uncover the enzymatic pathways involved in the regulation of homoarginine plasma concentration.

To face this challenge, a well characterized secondary outcome cohort of ischemic stroke patients was studied in view of a potential association with homoarginine (see Example 1 below). By applying genome-wide association (GWA) analysis, an association of homoarginine plasma concentration with certain types of genetic alterations was established (see Example 2 below). Finally, it was demonstrated in mice with a dysfunctional AGAT gene (AGAT^{-/-}), which were experimentally challenged with temporal occlusion of the middle cerebral artery (MCAO), that homoarginine levels are causally related to stroke outcome.

### EXAMPLE 1: Association of homoarginine with stroke outcome

For examining a potential association between the serum concentration of homoarginine and outcome in stroke patients, the Leeds Stroke Study was used. The recruitment and characteristics of patients in the Leeds Stroke Study have been fully described elsewhere (Carter et al. (2007), Stroke, 38(6): 1873-80). Briefly, European patients (n=609) with a clinical diagnosis of acute ischemic stroke were recruited from four hospitals in Leeds. Ischemic stroke was classified according to the Oxfordshire Community Stroke Project classification, as lacunar infarction (LACI), total and partial anterior circulation infarction (TACI, PACI), and posterior circulation infarction (POCI) (Ramford et al. (1991), Lancet 337: 1521-1526). Subjects were classified according to smoking history as current smokers, former smokers, or non-smokers, and a medical history of previous stroke or transient ischemic attack (TIA), ischemic heart disease (IHD) and peripheral vascular disease (PVD) was documented. Atrial fibrillation (AF) was established on clinical examination at stroke presentation and confirmed by 12-lead electrocardiogram (Carter et al. (2007), see above). Presence of diabetes and hypertension was determined from the case notes and current use of hypoglycemic and antihypertensive agents. Patients were flagged with the Office for National Statistics for notification of death, as previously described (Carter et al. (2007), see above). All subjects provided informed consent according to a protocol approved by the Leeds Teaching Hospitals Research Ethics Committee. Only patients who survived for longer than 30 days after the acute event with sufficient plasma available for analysis of homoarginine were included in the present examination (n=394).

Homoarginine in human plasma was determined with high performance-liquid chromatography (HPLC) and fluorescence detection after derivatization with o-phthalaldehyde as described previously (Teerlink et al. (2002), Anal Biochem. 303:131-7) and by liquid chromatography-tandem mass spectrometry (LC-MS/MS) after derivatization with butanolic hydrochloric acid as described in the below examples. Venous blood samples from stroke patients were also analysed for biochemical, haematological and haemostatic factors as previously described (Carter et al. (2007), see above). Estimated glomerular filtration rate (eGFR) was analysed according to the abbreviated MDRD equation for Caucasians: 186 x (Creat/88.4) - 1.154 x (Age) - 0.203 x (0.742 for females).

The primary outcome measure was all-cause long-term mortality after acute ischemic stroke. Associations between variables were assessed by Spearman correlation coefficients (rho), wherein a rho>0.2 was regarded clinically relevant. Differences between two unrelated groups were compared using unpaired Student's t-tests and between >2 groups by one-way ANOVA. Differences between categorical data were analysed by χ2 test. Univariate associations between quartiles of homoarginine and mortality were assessed using Kaplan-Meier survival analysis with significance determined using the log rank test. The association between homoarginine and mortality was determined using multivariate Cox regression analyses including variables previously shown to be independently associated with mortality in this cohort (Carter et al. (2007), see above), with data presented as hazard ratios (with 95% confidence intervals). Statistical analyses were performed using SPSS for Windows v12.0 (SPSS Inc.).

Median survival of the patients was followed for 7.4 years (range of 5.3 to 8.5 years). It was found that mean plasma concentrations of homoarginine in patients with acute ischemic stroke were higher in individuals who survived (n=163) as compared to those who died (n=231), i.e. 1.18 (95% CI: 1.11, 1.26) and 0.90 (0.84, 0.96) µmol/L, respectively (p<0.001). The traditional risk factors for stroke (previous stroke/TIA, atrial fibrillation, ischemic heart disease, and prevalent vascular disease) as well as older age) were more frequent in patients who died. Age and sex adjusted correlations were evident between homoarginine and L-arginine (rho=0.41), β-thromboglobulin (-0.32), fibrinogen (-0.24), serum creatinine (-0.202), eGFR (0.25), serum total cholesterol (0.25), and high sensitive CRP (-0.38, p<0.001 for all) as well as with von Willebrand Factor (-0.31, p<0.01).

All-cause mortality was significantly higher in patients with homoarginine in the lowest quartile and significantly decreased with increasing quartiles of homoarginine (Figure 1). In subgroup analysis, homoarginine was associated with stroke subtype, i.e. higher in small vessel disease (n=137, p<0.001), and lower in patients with atrial fibrillation (n=64, p=0.004). In univariate Cox regression analysis, stroke patients with low homoarginine were at risk for death. After adjustment for traditional risk factors, this association was still evident.

Result: It can be concluded from the above that homoarginine is related to the survival in patients after acute ischemic stroke. Homoarginine hence represents a common risk in cardiovascular ischemia, irrespective of coronary or cerebral localization of ischemia.

### EXAMPLE 2: Association of homoarginine with certain genotypes

To assess the genome-wide association (GWA) of homoarginine plasma concentration variability with potential alterations of the genotype a GWA analysis was performed in the population-based cohort of the Gutenberg Heart Study. The Gutenberg Heart Study (GHS) was initiated in 2006 to achieve a contemporary German sex-specific cardiovascular risk score. The study is a community-based, prospective cohort study including subjects aged 35 to 74 years from the city of Mainz and the district Mainz-Bingen (Wild et al. (2010), Circ Cardio-vasc Imaging; 3(5): 604-13). The sample is stratified according to gender (50% women) and decade of age. By December 2010, a total of 3,500 study participants with GWA data and biomarker measurements were eligible for the present investigation. Genotyping was performed on the Affymetrix Human SNP Array 6.0 platform. Extensive quality control (QC) analyses were performed before data were analyzed.

We performed a linear regression relating the trait to genotype dosage (0-2 copies of the minor allele) for each SNP, adjusting for age, sex, height, and weight. The association of the trait to the genotype was quantified by the regression slope (β), its standard error [SE(β)] and p-value. We selected an a priori genome-wide statistical significance threshold of 5*10⁻⁷ (The Wellcome Trust Case Control Consortium. Nature (2007), 447:661-678).

Only a single significant association of homoarginine plasma concentration among approximately 1 million genetic variants was found for the gene encoding L-arginine:glycine amidinotransferase (AGAT). We confirmed these discovery data by analysis of distinct AGAT SNPs in our cohort of ischemic stroke patients. Table 2 shows the associations and one-sided p-values for three representative single SNPs of the AGAT gene. These SNPs (rs10519022, rs9783731, and rs1145077) represent intron, downstream, and upstream regions of the AGAT gene in a haplotype view. Two out of these three SNPs, i.e. rs10519022 and rs1145077, along with the SNP rs128875 were selected for replicate analysis in the acute stroke cohort. Within this confirmatory analysis, all selected SNPs were associated with homoarginine plasma concentration variability (p=0.002, p=0.045, and p=0.001, respectively).

Result: The above observations clearly indicate that the AGAT gene encodes an enzyme which is involved in the synthesis of homoarginine in humans.

### EXAMPLE 3: Generation of AGAT^{-/-} and GAMT^{-/-} mice

To further elucidate the role of AGAT in vertebrates AGAT-deficient knockout mice were generated by homologous recombination in mouse embryonic stem cells. To this end, a targeting vector containing exons 3-7 was subcloned and exon 3 was disrupted by insertion of a loxP-flanked neomycin selection cassette. The linearized targeting vector was electroporated into embryonic stem (ES) cells, which were subjected to selection by geneticin (G418, Invitrogen, Karlsruhe, Germany). Homologous recombination was verified by Southern blot analysis of digested genomic ES cell DNA as described previously (Schmidt A, et al. (2004), D. Hum Mol Genet., 13:905-21). Two positive clones were expanded and microinjected into C57BL/6J mouse blastocysts, which were then transferred into pseudo-pregnant CBA/C57BL/6J females. Two of the chimeric mice that were mated gave rise to germ-line transmission of the disrupted allele. To excise the neomycin selection cassette from the targeted allele, AGAT^{+/-} were crossed with a cre deleter mouse line expressing cre recombinase in the germ line. Males and females with different genotypes from different litters were randomly intercrossed to obtain AGAT^{+/+}, AGAT^{+/-} and AGAT^{-/-} progeny. Genomic DNA from mouse-tail or ear biopsies was screened either by Southern analysis or multiplex polymerase chain reaction (PCR) following standard protocols.

A mouse model having a deficiency in the gene encoding the guanidinoacetate N-methyltransferase (GAMT) was obtained as described by Schmidt et al. (2004), Hum Mol Genet., 13:905-21. GAMT is the enzyme catalyzing the second step in creatine synthesis (see Fig. 4). GAMT^{-/-} mice were compared to AGAT^{-/-} mice in terms of guanidino acetate (GAA), creatine, homoarginine and AGAT expression.

Homoarginine in human plasma was determined with high performance-liquid chromatography (HPLC) and fluorescence detection after derivatization with o-phthalaldehyde (Teerlink et al. (2002), Anal Biochem, 303:131-7) and by liquid chromatography-tandem mass spectrometry (LC-MS/MS) after derivatization with butanolic hydrochloric acid. Homoarginine in mouse plasma was determined with LC-MS/MS after derivatization with butanolic hydrochloric acid. The concentrations of the guanidino compounds were determined using a Biotronic LC 5001 (Biotronik, Maintal, Germany) amino acid analyzer adapted for guanidino compound determination. The guanidino compounds were separated via a cation exchange column using sodium citrate buffers and were detected with the fluorescence ninhydrin method, which is described in detail elsewhere (Marescau et al. (1992), Metabolism., 41:526-32).

Results: In AGAT^{-/-} mice, no detectable AGAT protein expression was observed (see Fig. 2a). Also, creatine, GAA, and homoarginine were not detectable in comparison to wild type littermates (see Fig. 2b-d). AGAT^{-/-} animals develop a severe phenotype of muscle, growth, and mental retardation. When these animals are supplemented with creatine they develop normally and in line with wild type littermates. Despite supplementation of creatine in AGAT^{-/-} mice, guanidinoacetate and homoarginine levels remained undetectable (Fig. 2c-d).

GAMT^{-/-} mice showed a strong AGAT-overexpression (Fig. 2e). These mice furthermore exhibited increased homoarginine concentrations in serum and brain tissue as well as impaired creatine and ameliorated GAA (Fig. 2f-h).

### EXAMPLE 4: Model of ischemic stroke in AGAT^{-/-} and GAMT^{-/-} mice

In the next stage, a role of homoarginine for the outcome after acute ischemic stroke was experimentally examined. To this end, AGAT^{-/-} mice (with or without creatine supplementation) and GAMT^{-/-} mice were experimentally challenged by temporal occlusion of the middle cerebral artery (MCAO).

Temporary middle cerebral artery occlusion (MCAO) was achieved in mice (male, 20-25 g, 12-15 weeks) as previously described (see Choe et al. (2009), J Neurochem., 110:1766-73). After 24 h, mice were sacrificed using CO₂, cardiac blood samples were obtained and infarct sizes were quantified using triphenyltetrazolium chloride (TTC). A subgroup of animals was fed a special diet containing 1 % creatine ad libidum after ablactation (sniff, Germany). All investigators were blinded concerning genotypes and treatment. All experiments were approved by the local animal ethics committee (TV-Nr. 08/08 and 110/10).

Result: Temporary occlusion for 30 minutes resulted in 50% larger stroke area volumes in AGAT^{-/-} mice (n = 7/6, p<0.01) as compared to wild-type littermates, as well as aggravated neurological impairment (Fig. 3a-c). Creatine supplementation did not influence stroke size, despite sufficient cerebral creatine replenishment as visualized by MR-spectroscopy (data not shown). To account for underlying mechanisms involved in the stroke phenotype of AGAT^{-/-}mice, we analyzed brain morphology, cerebral nitric oxide metabolism, vascular function, oxidative stress, and hemostasis. Cortical expression analysis of genes related to NO metabolism revealed no differences between wild-type and AGAT^{-/-} mice nor histological abnormalities in cortex or hippocampus. Also, endothelium-dependent and -independent vasodilatation of the aorta was not different. Urinary excretion of 8-iso prostaglandin F2a, a marker of oxidative stress, was similar between both groups. Analysis of hemostasis did not show any differences except a prolonged prothrombin time in AGAT^{-/-} mice.

To provide evidence that declined stroke outcome was contingent on homoarginine, GAMT^{-/-}mice were subjected to MCAO. These animals are also deficient in creatine but not in homoarginine biosynthesis. They did not show lager stroke volumes as compared to wild type littermates after MCAO (Fig. 3d-g). Moreover, stroke volume and neurological score were significantly improved, most likely due to elevated homoarginine levels in these animals (data not shown). From these observations, it can be concluded that homoarginine is causally related to stroke outcome independent of the genetic relation of AGAT to energy metabolism.

### EXAMPLE 5: Measuring homo arginine concentrations in plasma

For the determination of the basal plasma concentrations of L-homoarginine, 136 apparently healthy volunteers provided informed consent and plasma samples.

To analyze the plasma samples with LC MS/MS by a stable isotope dilution assay, aliquots of human EDTA, heparin, citrate or serum plasma were used. Aliquots were 25 µL, but other volumes may be used instead, i.e. 10 µL, 20 µL, 50 µL, 100µL, or 250 µL. For protein precipitation, 96-well 0.20-µm microfiltration plates were used. Each well of the microfiltration plate was filled with 100 µl of internal standard solved in methanol. L-[¹³C6]-homoarginine was used as internal standard at a final concentration of 10 µmol/L plasma sample. Other internal standards can be used like L-[²H₆]-homoarginine, L-[¹⁵N₂]-homoarginine, L-[²H₂]-arginine, L-[²H₄]-arginine, L-[²H₇]-arginine, L-[¹³C₆]-arginine, or L-[¹⁵N₂]-arginine. Other final concentrations of the internal standard may be used like 2, 5, or 20 µmol/L. The 96-well filtration plate had to be placed on top of a 96-well polypropylene plate. Subsequently, each well was piled with a 25-µl sample aliquot. Proteins were precipitated quantitatively by shaking the 96-well plates for 15 min using an orbital shaker. To separate analytes from precipitated proteins, the microfiltration plates on top of the polypropylene plates were centrifuged for 10 min at 800 x g (Eppendorf centrifuge 5810R, 2000 rpm, Hamburg, Germany). After centrifugation, the eluates in the polypropylen plate were dried by heating at 85°C on a 96-well aluminium block for 30 min.

Compounds were derivatized to their butyl ester derivatives by adding 100 µl of 1 M HCl in 1-butanol. The plates were coated with acetate foil, and the 96-well polypropylen plates were heated at 65°C for 30 min. The plates were centrifuged for 1 min at 800 x g and the acetate foil was removed. The derivatization reagent was dried by heating at 85°C for 30 min. The samples were resolved in 100 µL of methanol:water (25:75) containing 0.1% ammonium formate. The pH was adjusted to pH 5 with formic acid. Samples were transferred in another 96-well 0.20-µm microfiltration plate on top of a polypropylene plate and centrifuged as described above. The calibrators and the quality controls were treated exactly the same as the plasma samples. Afterwards, polypropylene plates were placed in a CTC PAL autosampler (Varian, Palo Alto, CA, USA), and 20-µl aliquots were subjected to further analysis.

LC_MS/MS analyses were performed on a Varian 1200L Triple Quadrupole MS equipped with two Varian ProStar model 210 HPLC pumps (Varian, Lake Forest, CA, USA). Separation of analytes from major matrix components was achieved with a Polaris C18-Ether column (Varian) using an elution gradient of acetonitrile-methanol (50/50, v/v) containing 0.1% aqueous formic acid (0:00 min 95/5 (v/v) - 0:30 95/5 - 2:00 50/50 - 2:01 95/5 - 4:00 95/5) at 30°C, with a flow rate of 0.3 mL /min.

Nitrogen was used as the nebulizing and drying gas (250°C) at 90 and 180 1/h, respectively. For positive electrospray ionisation (ESI+) the needle and shield voltage were set at 5000 and 600 V, respectively. For quantitative analyses the analytes were detected by means of characteristic product ions formed from protonated molecules by collision-induced dissociation (CID) using the multiple reaction monitoring (MRM) mode after fragmentation with argon (2 Pa): m/z 245 to 211 (collision energy 14 eV) for L-homoarginine, and m/z 251 to 217 (14 eV) for L-[¹³C₆]-homoarginine. Dwell time was set to 0.5 s for L-homoarginine and L-[¹³C₆]-homoarginine.

Calibration standards were prepared in the same manner as plasma samples. The calibration curve was constructed from integrated chromatograms using peak area ratios of L-homoarginine to the internal standard L-[¹³C₆]-homoarginine. The lower limit of quantification (LLOQ), intraassay and interassay accuracy and precision of L-homoarginine were determined by calculating the recovery of added L-homoarginine to dialyzed human plasma. For LLOQ and intraassay accuracy and precision nine different concentrations (n=4, each) of L-homoarginine, i.e. 0.05, 0.1, 0.25, 0.5, 1, 5, 10, 25, and 50 µM were added to dialyzed human plasma. Interassay accuracy and precision were performed by adding two different concentrations (2 and 5 µM, n=2, each) on seven separate 96-well plates. The limit of detection (LOD) was estimated according to FDA guidelines (Guidance for the industry - Bioanalytical Method Validation, US Department of Health and Human Services, Food and Drug Administration, Centre for drug Evaluation and Research, May 2001).

Result: The limit of detection (LOD) for L-homoarginine was determined as 5 nmol/L (S/N=9±5). Average precision (R.S.D.) for a concentration range from 0.1 to 50 µmol/L was 7.4±4.5% (mean±SD). The observed bias for all added concentrations was +1.1±7.4%. The LLOQ based on this analysis was 0.1 µmol/L (n=4). Linear regression analysis between peak area ratios (y) and added (x) concentrations yielded slope and y-axis intercept of 0.15 and 0.01 (r2=0.99). The mean interassay variability for 2 and 5 µmol/L was 7.5±2.0%.

The mean plasma concentration of L-homoarginine in healthy humans was found to be in the range of 2.5±1.0 µmol/L.

## Claims

1. Method for predicting the all-cause mortality risk of a test subject, comprising
(a) detecting in a sample from said test subject the expression level of the L-arginine:glycine amidinotransferase (AGAT) gene;
(b) comparing the expression level of step (a) to the expression level of said gene in a reference subject;
wherein detection of an expression level of the AGAT gene which is significantly decreased in the test subject relative to the expression level in the reference subject indicates that the test subj ect has an increased mortality risk.

2. Method of claim 1, wherein the expression level of the AGAT gene in step (a) is detected by quantitative RT-PCR, TaqMan^{™} RT-PCR, real-time RT-PCR, or Northern-Blot analysis.

3. Method of any of claims 1-2, wherein the expression level of the AGAT gene in step (a) is decreased by at least 2-fold, at least 5-fold or at least 10-fold relative to the expression level in the reference subject.

4. Method for predicting the all-cause mortality risk of a test subject, comprising
(a) detecting in a sample from said test subject the activity of the L-arginine:glycine amidinotransferase (AGAT) enzyme;
(b) comparing said activity of step (a) to the activity of said enzyme in a reference subj ect;
wherein detection of an activity of the AGAT enzyme which is significantly decreased in the test subject relative to the activity in the reference subject indicates that the test subj ect has an increased mortality risk.

5. Method of claim 4, wherein the activity of the AGAT enzyme in step (a) is detected by ELISA, mass spectrometric methods or immunohistochemical methods.

6. Method of any of claims 4-5, wherein the activity of the AGAT enzyme in step (a) is decreased by at least 2-fold, at least 5-fold or at least 10-fold relative to the activity in the reference subj ect.

7. Method for predicting the all-cause mortality risk of a test subject, comprising
(a) detecting in a sample from said test subject the concentration of homoarginine;
(b) comparing the concentration of step (a) to the concentration of homoarginine in a sample of a reference subject;
wherein detection of a homoarginine concentration which is significantly decreased in the test subject relative to the concentration in the reference subject indicates that the test subject has an increased mortality risk.

8. Method of claim 7, wherein the sample is a blood plasma sample.

9. Method of any of claims 7-8, wherein the concentration of homoarginine in the sample is detected by immunoassay methods or chromatographic methods.

10. Method for predicting the all-cause mortality risk of a test subject, comprising detecting in a biological sample a genetic alteration in the genome of said test subject, wherein said genetic alteration is associated with a decreased expression of the AGAT gene or with a decreased activity of the AGAT enzyme; wherein the detection of said genetic alteration indicates that the test subj ect has an increased mortality risk.

11. Method of claim 10, wherein said detection of a genetic alteration comprises the detection of at least one single nucleotide polymorphism (SNP).

12. Method of any of claims 10-11, wherein said single nucleotide polymorphism occurs in position 27 of any of the sequences depicted in SEQ ID NOs:1-15.

13. Method of any of the preceding claims, wherein the all-cause mortality is the all-cause mortality after having experienced a cardiovascular event, such as a stroke.

14. *In vitro* method for identifying a pharmaceutically active compound which decreases the all-cause mortality risk of a subject, comprising
(a) contacting a candidate compound with a cell that expresses the L-arginine:glycine amidinotransferase (AGAT) gene;
(b) detecting whether said candidate compound alters the expression level of the AGAT gene;
wherein an increase of the expression level of the AGAT gene indicates a pharmaceutically active compound.

15. *In vitro* method for identifying a pharmaceutically active compound which decreases the all-cause mortality risk of a subject, comprising
(a) contacting a candidate compound with a L-arginine:glycine amidinotransferase (AGAT) enzyme;
(b) detecting whether said candidate compound alters the activity of the AGAT enzyme;
wherein an increase of activity of the AGAT enzyme indicates a pharmaceutically active compound.

16. Compound which is effective in increasing the expression of the AGAT gene and/or the activity of AGAT enzyme for use in a method of decreasing the mortality risk of a subj ect.

17. Composition comprising homoarginine or a derivative of homoarginine for use in a method of decreasing the all-cause mortality risk of a subject.
